# EUROPEAN PATENT SPECIFICATION

(11) **EP 2 545 887 B1**
(45) Date of publication and mention of the grant of the patent: **10.08.2016**
(21) Application number: 11762675.4
(22) Date of filing: 24.03.2011
(51) Int. Cl.: A61F 2/95, A61F 2/962, A61F 2/966

(54) **STENT DELIVERY SYSTEM**
STENTTRÄGERSYSTEM
SYSTÈME DE POSE D'ENDOPROTHÈSE

(30) Priority: 30.03.2010 JP 2010077679
(43) Date of publication of application: 16.01.2013
(73) Proprietor: Terumo Kabushiki Kaisha, Tokyo 151-0072 (JP)
(72) Inventor: SUGIMOTO Ryota, Ashigarakami-gun Kanagawa 259-0151 (JP); KITAOKA Takashi, Ashigarakami-gun Kanagawa 259-0151 (JP)
(74) Representative: Prüfer & Partner mbB Patentanwälte · Rechtsanwälte
(86) International application number: PCT/JP2011/057209
(87) International publication number: WO 2011/122444

(56) References cited:
- EP-A1- 1 374 801
- EP-A2- 0 696 447
- EP-A2- 1 982 677
- WO-A1-2008/034793
- WO-A1-2010/078352
- JP-A- 11 514 536
- JP-A- 2001 500 403
- JP-A- 2008 501 442
- JP-A- 2008 508 936
- US-A1- 2005 033 403
- US-A1- 2005 149 159
- US-A1- 2007 233 224
- US-A1- 2009 024 133
- US-A1- 2009 157 162
- US-A1- 2009 192 584

## Description

### Technical Field

The present invention relates to a stent delivery system for use in improvement of a stenosis or an occluded part generated in a living-body lumen such as blood vessel, bile duct, trachea, esophagus, or urethra.

### Background Art

A stent delivery system, in general, has a stent for improving a stenosis or an occluded part. The stent is generally a tubular medical device which, for treating various diseases arising from stenosis or occlusion of a blood vessel or other living-body lumen, is used to dilate the stenosed or occluded part and indwelled there to secure an inner cavity.

The description will be made below taking a blood vessel as an example, which is a non-restrictive example.

A stent is a device which is small in diameter at the time of insertion into a living body from the outside, and which is expanded in a targeted stenosis or occluded part to increase in diameter and to maintain the lumen as it is.

In general, a stent has a cylindrical body formed from by processing metallic wires or a metallic pipe. The stent is mounted to a catheter or the like in a radially reduced state, is inserted into a living body, is expanded in a target part (stenosis or occluded part) by some method, and is fixed in secure contact with the inner wall of the lumen, thereby maintaining the lumen shape. Stents are classified, by function and indwelling method, into self-expandable stents and balloon-expandable stents. A balloon-expandable stent does not have an expanding function in itself. The stent mounted on a balloon is inserted into a target part, then the balloon is inflated, and the stent is expanded (plastically deformed) by dilation force of the balloon, whereby the stent is fixed in secure contact with the inner surface of the target lumen. This type of stent needs the just-mentioned stent-expanding operation. On the other hand, a self-expandable stent is provided with an expanding function of its own. The self-expanding stent is inserted into a living body in a radially reduced state, and is opened up in a target portion to spontaneously return into its original expanded state, thereby being fixed in secure contact with the inner wall of the lumen and maintaining the lumen shape.

The purpose of indwelling of a stent nowadays is mostly to return a blood vessel stenosed or occluded for some reason into its original patent state; in fact, most of the stents are used mainly for prevention or restraining of re-stenosis which might occur after such a procedure as PTCA. In recent years, for suppressing the probability of re-stenosis more assuredly, drug-eluting stents carrying thereon a drug such as immunosuppressant or carcinostatic agent have also been used, and their effects have been publicly known.

Many of the self-expandable stents are used in peripheral areas such as inferior limb or carotid artery, and include, for example, stents having a form as shown in Patent Document 1 (JP-T-H11-505441, WO96/26689).

In addition, Patent Document 2 (JP-T-2007-504897, WO2005/032614) discloses a system for delivery and deployment of a medical device (stent) into a patient's body, which has a delivery catheter including an inner catheter member having a region for attaching the medical device and an outer restraining member coaxially fitted over the inner catheter member and the medical device. In this delivery system, the outer restraining member is capable of movement in an axial direction relative to the inner catheter member, and a control handle which has a rotatable thumbwheel connected to a retraction mechanism is provided. The inner catheter member has a proximal end portion attached to the control handle, and the outer restraining member has a proximal end portion attached to the retraction mechanism. With the thumbwheel rotated, a rectilinear motion of the retraction mechanism is induced, an outer restraining member sheath is retracted toward the proximal end, and the medical device is exposed with the inner catheter member kept stationary.

### Prior Art Documents

### Patent Documents

Patent Document 1: JP-T-H11-505441 (WO96/26689)
Patent Document 2: JP-T-2007-504897 (WO2005/032614)
Patent Document 3: US 2009/0024133 A1

### Summary of Invention

### Technical Problems

In the stent delivery system using a self-expandable stent as in Patent Document 1, the self-expanding property possessed by the stent makes it difficult to position the stent at the time of stent indwelling compared with the case of a balloon-expandable stent. Further, a jumping phenomenon may occur in which the stent jumps out from the stent delivery system. If this phenomenon occurs, the stent would be placed at a position deviated from the planned placement position. In addition, there is a case where, after the stent is discharged to a certain extent during the stent indwelling procedure, readjustment of the indwelling position of the stent is needed. In the system as described in Patent Document 1, however, re-accommodation of the stent into the stent delivery system is difficult to achieve.

In the stent delivery system disclosed in Patent Document 2, the operability of the outer restraining member for releasing the stent is good. Even in the stent delivery system in Patent Document 2, however, re-accommodation of the stent into the stent delivery system is difficult to perform.

Patent document 3 discloses a delivery device comprising a gear and pulley mechanism and a set of sheaths to deploy a prosthesis into a body lumen, additionally allowing to re-sheath the prosthesis back into the catheter.

In view of the foregoing, it is an object of the present invention to provide a stent delivery system using a self-expandable stent, in which a stent-releasing operation can be performed favorably, a stent can be re-accommodated into a stent-accommodating tube body even after the stent is exposed to a certain extent from the stent-accommodating tube body, and the operation of accommodating the stent into the stent-accommodating tube body is easy to carry out.

### Technical Solution

The above object is attained by the stent delivery device disclosed in claim 1.

In particular a stent delivery system comprising: a stent delivery system main body including a stent having a multiplicity of side-wall openings, formed in a roughly cylindrical shape, compressed toward a center axis thereof at the time of insertion into a living body, and capable of being restored into its pre-compression shape by expanding outward at the time of indwelling in the living body, an inner tube body having a guide wire lumen, and a stent-accommodating tube body accommodating said stent in a distal end portion thereof, said stent being so disposed as to cover a distal end portion of said inner tube body, and said stent being releasable by moving said stent-accommodating tube body in a proximal direction relative to said inner tube body; and an operation unit disposed at a proximal end portion of the stent delivery system main body and having a moving mechanism for moving said stent-accommodating tube body, wherein said inner tube body includes a distal-side tube having said guide wire lumen, and a proximal-side tube connected to a proximal end side of said distal-side tube and penetrating said stent-accommodating tube body, said operation unit includes a housing, a shaft-like rack member accommodated in said housing and fixed to a proximal end of said stent-accommodating tube body, an operation rotary roller having a working gear wheel which engages with teeth of said rack member so as to move said rack member within said housing, and a connector which is fixed to a proximal end portion of said proximal-side tube protruding beyond the proximal end of said stent-accommodating tube body fixed to said rack member and which is held by said housing, and said stent delivery system has a stent-holding function for releasably holding said stent and for enabling re-accommodation of said stent into said stent-accommodating tube body by forward movement of said stent-accommodating tube body after partial exposure of said stent from said stent-accommodating tube body, said stent can be released from said stent-accommodating tube body by movement of said rack member toward said connector by rotation of said operation rotary roller in a predetermined direction, and, after partial exposure of said stent from said stent-accommodating tube body, said stent can be re-accommodated into said stent-accommodating tube body by moving said rack member within said housing in a direction opposite to a direction toward said connector through rotating said operation rotary roller in a direction reverse to the predetermined direction.

### Advantageous Effects

The stent delivery system according to the present invention includes: the stent delivery system main body including the stent, the inner tube body, and the stent-accommodating tube body in the distal end portion of which the stent is accommodated; and the operation unit having the moving mechanism which is disposed at the proximal end portion of the stent delivery system main body and which is for moving the stent-accommodating tube body. The inner tube body includes the distal-side tube having the guide wire lumen, and the proximal-side tube connected to the proximal end side of the distal-side tube. The operation unit includes: the housing; the shaft-like rack member accommodated in the housing and fixed to the proximal end of the stent-accommodating tube body; the operation rotary roller which has the working gear wheel that engages with the teeth of the rack member so as to move the rack member within the housing; and the connector which is fixed to the proximal end portion of the proximal-side tube protruding beyond the proximal end of the stent-accommodating tube body fixed to the rack member and which is held by the housing. In addition, the stent delivery system has the stent-holding function for releasably holding the stent and for enabling re-accommodation of the stent into the stent-accommodating tube body by forward movement of the stent-accommodating tube body after partial exposure of the stent from the stent-accommodating tube body. Further, the stent can be released from the stent-accommodating tube body by movement of the rack member toward the connector by rotation of the operation rotary roller in the predetermined direction. In addition, after partial exposure of the stent from the stent-accommodating tube body, the stent can be re-accommodated into the stent-accommodating tube body by moving the rack member within the housing in the direction opposite to the direction toward the connector through rotating the operation rotary roller in the direction reverse to the predetermined direction.

In the stent delivery system according to the present invention, by rotation of the operation rotary roller in the predetermined direction, the shaft-like rack member is moved within the housing toward the connector, whereby the stent can be released from the stent-accommodating tube body. Therefore, a stent-releasing operation is easy to carry out. Further, after partial exposure of the stent from the stent-accommodating tube body, the stent can be re-accommodated into the stent-accommodating tube body by moving the shaft-like rack member within the housing in the direction opposite to the direction toward the connector through rotating the operation rotary roller in the direction reverse to the predetermined direction. Therefore, it is possible, even after the stent is exposed from the stent-accommodating tube body to a certain extent, to re-accommodate the stent into the stent-accommodating tube body. Thus, re-placement of the stent can be performed. In addition, the operation of accommodating the stent into the stent-accommodating tube body is easy to conduct, since it is only necessary to rotate the roller.

Besides, where a configuration is adopted in which the proximal-side tube has a lumen a distal end portion of which opens in the stent-accommodating tube body and which provides communication to the proximal end of the proximal-side tube and in which liquid can be injected into the stent delivery system from the connector by using the lumen in the proximal-side tube, priming of the inside of the distal end portion of the stent-accommodating tube body is easy to carry out. Further, liquid (for example, a drug) can be ejected from the distal end of the stent-accommodating tube body.

### Brief Description of Drawings

[FIG. 1] FIG. 1 is a partly omitted external appearance view of a stent delivery system as an embodiment of the present invention.
[FIG. 2] FIG. 2 is an enlarged view of a distal end portion of the stent delivery system shown in FIG. 1.
[FIG. 3] FIG. 3 is an enlarged longitudinal sectional view of the distal end portion of the stent delivery system shown in FIG. 1.
[FIG. 4] FIG. 4 is a partly omitted enlarged external appearance view of an inner tube body (including a stent) of the stent delivery system shown in FIG. 1.
[FIG. 5] FIG. 5 is an illustration for explaining the vicinity of the distal end portion of the stent delivery system shown in FIG. 1.
[FIG. 6] FIG. 6 is a partly omitted enlarged sectional view of the distal end portion of the stent delivery system shown in FIG. 1.
[FIG. 7] FIG. 7 is an illustration for explaining an internal structure of the vicinity of an intermediate portion of the stent delivery system shown in FIG. 1.
[FIG. 8] FIG. 8 is a front view of an example of an in-vivo indwelling stent for use in the stent delivery system according to the present invention.
[FIG. 9] FIG. 9 is a development view of the in-vivo indwelling stent of FIG. 8.
[FIG. 10] FIG. 10 is an enlarged view of a proximal-end-side connection section of the in-vivo indwelling stent of FIG. 8.
[FIG. 11] FIG. 11 is a sectional view taken along a line A-A of FIG. 10.
[FIG. 12] FIG. 12 is an illustration for explaining an internal structure of an operation unit of the stent delivery system according to the present invention.
[FIG. 13] FIG. 13 is an enlarged front view of the operation unit of the stent delivery system according to the present invention.
[FIG. 14] FIG. 14 is a plan view of the operation unit of the stent delivery system shown in FIG. 13.
[FIG. 15] FIG. 15 is an illustration for explaining the internal structure of the operation unit of the stent delivery system according to the present invention.
[FIG. 16] FIG. 16 is an illustration for explaining the internal structure of the operation unit of the stent delivery system according to the present invention.
[FIG. 17] FIG. 17 is an illustration for explaining the internal structure of the operation unit of the stent delivery system according to the present invention.
[FIG. 18] FIG. 18 is an illustration for explaining an operation of the stent delivery system according to the present invention.
[FIG. 19] FIG. 19 is an illustration for explaining the operation of the stent delivery system according to the present invention.
[FIG. 20] FIG. 20 is an illustration for explaining the operation of the stent delivery system according to the present invention.
[FIG. 21] FIG. 21 is an illustration for explaining the operation of the stent delivery system according to the present invention.
[FIG. 22] FIG. 22 is an illustration for explaining the operation of the stent delivery system according to the present invention.
[FIG. 23] FIG. 23 is an enlarged longitudinal sectional view of a distal end portion of a stent delivery system as another embodiment of the present invention.
[FIG. 24] FIG. 24 is a development view of another example of the in-vivo indwelling stent for use in the stent delivery system according to the present invention.
[FIG. 25] FIG. 25 is an illustration for explaining a stent delivery system in which the in-vivo indwelling stent of FIG. 24 is used.

### Modes for Carrying Out the Invention

A stent delivery system according to the present invention will be described referring to an embodiment shown in the drawings.

A stent delivery system (in other words, a body organ lesion improving instrument) 1 according to the present invention includes a stent delivery system main body 2 and an operation unit 6 disposed at a proximal end portion of the stent delivery system main body 2. The stent delivery system main body 2 includes: a stent 10 having a multiplicity of side-wall openings, formed in a roughly cylindrical shape, compressed toward a center axis at the time of insertion into a living body, and capable of being restored into its pre-compression shape by expanding outward at the time of indwelling in the living body; an inner tube body 3 having a guide wire lumen 61; and a stent-accommodating tube body (stent-accommodating member) 5 which accommodates the stent 10 in a distal end portion thereof. In the stent delivery system main body 2, the stent 10 is so disposed as to cover a distal end portion of the inner tube body 3, and the stent 10 is releasable by moving the stent-accommodating tube body 5 in a proximal direction relative to the inner tube body 3. The operation unit 6 has a moving mechanism for moving the stent-accommodating tube body 5.

In addition, the inner tube body 3 includes a distal-side tube 31 having the guide wire lumen 61, and a proximal-side tube 34 connected to a proximal end side of the distal-side tube 31.

Besides, the operation unit 6 includes: a housing 40; a shaft-like rack member 43 accommodated in the housing 40 and fixed to a proximal end of the stent-accommodating tube body 5 (specifically, a proximal tube 22); an operation rotary roller 50 having a working gear wheel 54 which engages with teeth 66 of the rack member 43 and which is for moving the rack member 43 within the housing 40; and a connector 46 which is fixed to a proximal end portion of the proximal-side tube 34 penetrating the stent-accommodating tube body 5 (specifically, the proximal tube 22) fixed to the rack member 43 and protruding beyond the proximal end of the stent-accommodating tube body 5 and which is held by the housing 40.

In addition, the stent delivery system 1 has a stent-holding function 35, 36 which releasably holds the stent 10, and which, after partial exposure of the stent 10 from the stent-accommodating tube body 5, enables re-accommodation of the stent 10 into the stent-accommodating tube body 5 by forward movement of the stent-accommodating tube body 5. Furthermore, with the operation rotary roller 50 rotated in a predetermined direction, the shaft-like rack member 43 is moved within the housing 40 toward the connector 46, whereby the stent 10 can be released from the stent-accommodating tube body 5. In addition, with the operation rotary roller 50 rotated in a direction reverse to the predetermined direction after partial exposure of the stent 10 from the stent-accommodating tube body 5, the shaft-like rack member 43 is moved within the housing 40 in a direction reverse to the direction toward the connector 46, whereby the stent 10 can be re-accommodated into the stent-accommodating tube body 5.

Besides, in the stent delivery system 1 in this embodiment, the proximal-side tube 34 of the inner tube body 3 has a lumen 38 a distal end portion of which opens in the stent-accommodating tube body and which provides communication to a proximal end thereof. Incidentally, the proximal-side tube 34 is connected to a proximal end side of the distal-side tube 31 through a connection member. In addition, in the stent delivery system 1 in this embodiment, liquid can be injected into the stent delivery system from the connector 46 by using the lumen 38 in the proximal-side tube 34.

The stent delivery system 1 according to the present invention includes the stent delivery system main body 2 and the operation unit 6 disposed at the proximal end portion of the stent delivery system main body 2.

In addition, the stent delivery system 1 in this embodiment shown in the drawings includes: the stent 10 having the multiplicity of side-wall openings, formed in a roughly cylindrical shape, compressed toward the center axis at the time of insertion into a living body, and capable of being restored into its pre-compression shape by expanding outward at the time of indwelling in the living body; the inner tube body 3 having the guide wire lumen 61; and the stent-accommodating tube body 5 which accommodates the stent 10 in the distal end portion thereof. The stent 10 is so disposed as to cover the distal end portion of the inner tube body 3.

Besides, the stent delivery system 1 in the embodiment shown in the drawings includes: the stent 10 capable of being restored into its pre-compression shape by expanding outward at the time of indwelling in the living body; the stent-accommodating tube body 5 which accommodates the stent 10 in the distal end portion thereof; and the inner tube body 3 which is slidably passed through the inside of the stent-accommodating tube body 5 and by which the stent 10 is released via the distal end of the stent-accommodating tube body 5. The stent 10 has a distal end portion oriented toward the distal end of the stent-accommodating tube body 5, and a proximal end portion oriented toward the proximal end of the stent-accommodating tube body 5. Further, the stent 10 does not substantially have any bent free end which is at least oriented toward the proximal end, other than the proximal end portion thereof. After a distal-side portion of the stent 10 is exposed from the stent-accommodating tube body 5, the exposed portion can be re-accommodated into the stent-accommodating tube body 5 by moving the stent-accommodating tube body 5 in the distal direction. The stent delivery system 1 has the guide wire lumen 61 of which one end opens at the distal end of the stent delivery system, and the other end opens on the proximal side relative to a stent-accommodating part of the stent-accommodating tube body 5.

The stent delivery system main body 2 includes: the stent 10; the stent-accommodating tube body 5 which accommodates the stent 10 in the distal end portion thereof; and the inner tube body 3 slidably passed through the inside of the stent-accommodating tube body 5.

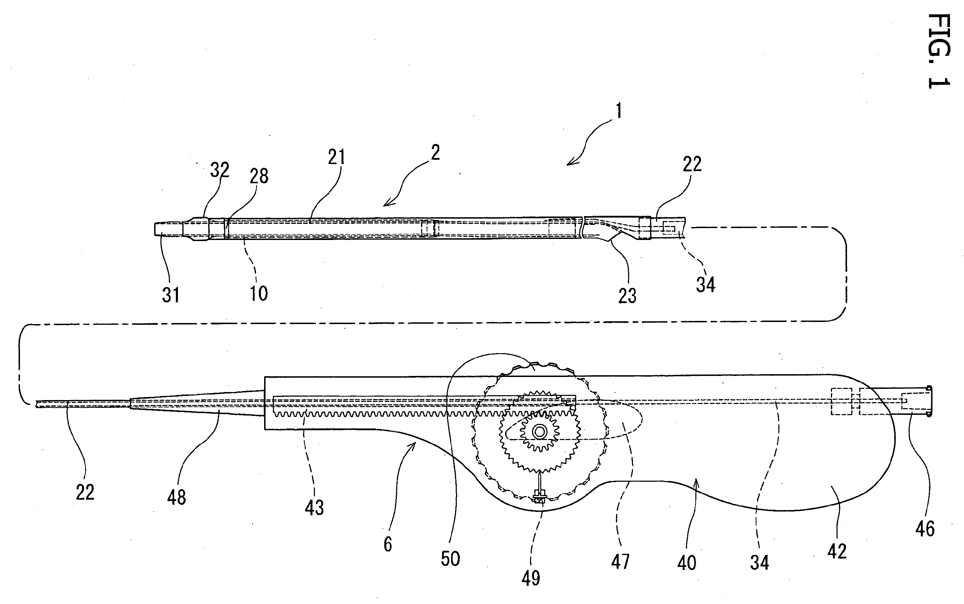

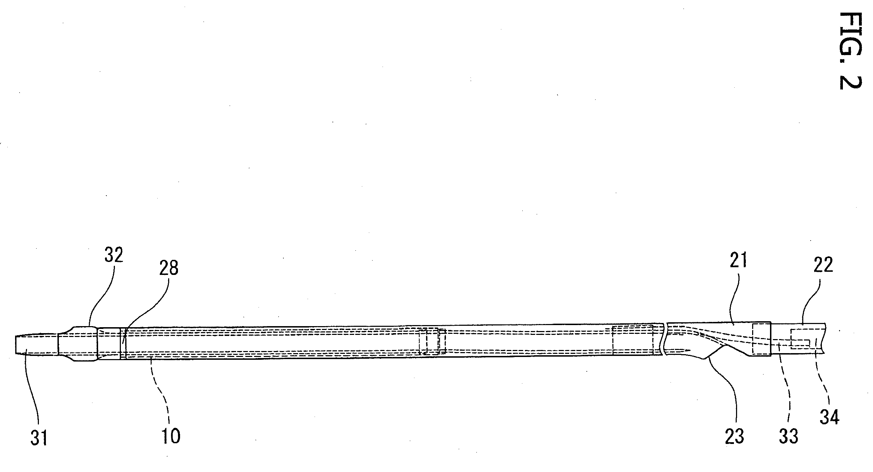

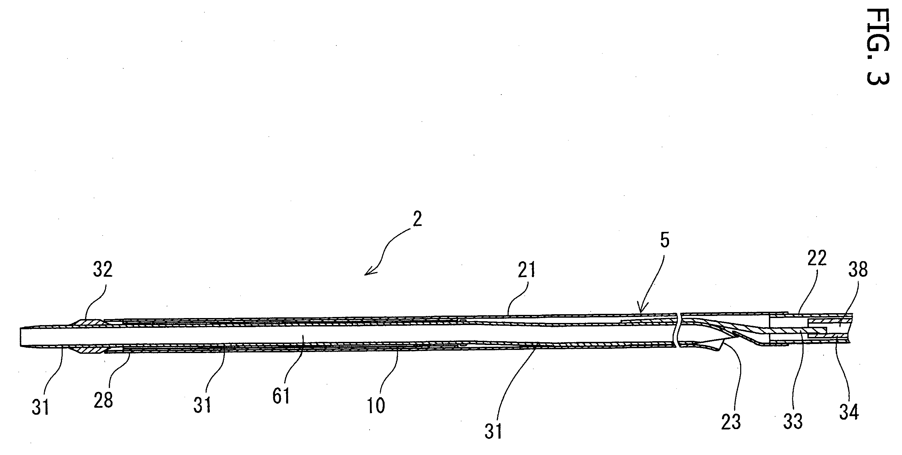

As shown in FIGS. 1 to 7, the stent-accommodating tube body 5 includes a distal tube 21, and the proximal tube 22 fixed to a proximal end of the distal tube 21.

The distal tube 21 is a tubular body, which opens at a distal end and the proximal end thereof. The distal opening functions as a release port for the stent 10 when the stent 10 is indwelled in a target portion of a lumen. The stent 10 is released via the distal opening, whereby it is relieved from a stress load, and expands to be restored into its pre-compression shape. A distal end portion of the distal tube 21 is the stent-accommodating part for accommodating the stent 10 in the inside thereof. In addition, the distal tube 21 has a side hole 23 disposed on the proximal side relative to the stent-accommodating part. The side hole 23 is a hole for leading out a guide wire therethrough to the exterior.

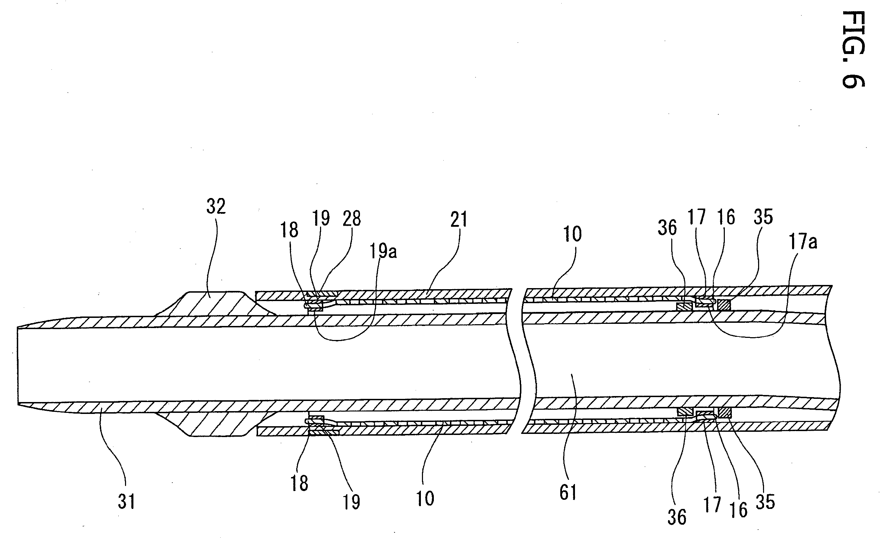

Besides, preferably, a radiopaque marker 28 is disposed at the distal end portion of the distal tube 21. As shown in FIG. 6, the stent 10 is accommodated in the distal tube 21 in such a manner that the position of the distal end thereof substantially coincides with the position of a distal end of the radiopaque marker 28. The radiopaque marker 28 is preferably formed in a tubular shape, from a radiopaque material. As a material for forming the radiopaque marker, one element (simple substance) or two or more elements (alloy) selected from an element group consisting of iridium, platinum, gold, rhenium, tungsten, palladium, rhodium, tantalum, silver, ruthenium, and hafnium can be used suitably.

In addition, the proximal tube 22 is a tube body having a lumen penetrating it from the distal end to the proximal end thereof. Of the proximal tube 22, the distal end is fixed to the proximal end of the above-mentioned distal tube 21, and a proximal end portion is fixed to the rack member 43 accommodated in the operation unit 6 which will be described later.

The outside diameter of the distal tube 21 is preferably 0.5 to 4.0 mm, particularly preferably 0.8 to 2.0 mm. Besides, the inside diameter of the distal tube 21 is preferably 0.2 to 1.8 mm. The length of the distal tube 21 is preferably 50 to 500 mm, particularly preferably 100 to 300 mm.

The outside diameter of the proximal tube 22 is preferably 0.3 to 4.0 mm, particularly preferably 0.5 to 1.0 mm. In addition, the inside diameter of the proximal tube 22 is preferably 0.1 to 1.0 mm. The length of the proximal tube 22 is preferably 500 to 4,000 mm, particularly preferably 800 to 2,000 mm.

Materials for forming the distal tube 21 and the proximal tube 22 are selected taking into account physical properties (flexibility, hardness, strength, sliding property, anti-kinking property, stretchability) required of the tubes. Examples of the preferable materials include stainless steel, superelastic metal, polyethylene, polypropylene, nylon, polyethylene terephthalate, fluoro polymer such as PTFE or ETFE, and thermoplastic elastomer. The thermoplastic elastomer is appropriately selected from nylon-based ones (e.g., polyamide elastomer), urethane-based ones (e.g., polyurethane elastomer), polyester-based ones (e.g., polyethylene terephthalate elastomer), and olefin-based ones (e.g., polyethylene elastomer, polypropylene elastomer).
In addition, the distal tube 21 is more flexible than the proximal tube 22. Such a setting ensures good operability.
Furthermore, an outer surface of the stent-accommodating tube body 5 (the distal tube 21 and the proximal tube 22) is preferably subjected to a treatment for causing the outer surface to exhibit lubricity. Examples of such a treatment include a method in which the outer surface is coated with a hydrophilic polymer such as poly (2-hydroxyethyl methacrylate), polyhydroxyethyl acrylate, hydroxypropyl cellulose, methyl vinyl ether-maleic anhydride copolymer, polyethylene glycol, polyacrylamide, polyvinylpyrrolidone, and dimethylacrylamide-glycidyl methacrylate copolymer, or a method in which the hydrophilic polymer is fixed onto the outer surface. In addition, an inner surface of the distal tube 21 may be coated with the above-mentioned hydrophilic polymer or the hydrophilic polymer may be fixed onto the inner surface, for ensuring good slidability of the inner surface in relation to the stent 10 and the inner tube body 3.

As shown in FIGS. 1 to 7, the inner tube body 3 includes: the distal-side tube 31 of which a distal end portion protrudes beyond the distal end of the stent-accommodating tube body 5; the proximal-side tube 34; a wire-formed member 33 for interconnecting a proximal end portion of the distal-side tube 31 and a distal end portion of the proximal-side tube 34; and the connector 46 fixed to the proximal end of the proximal-side tube 34.

Besides, in this embodiment, the inner tube body 3 has a proximal-side opening of the guide wire lumen which opens in a side portion on the proximal side relative to the stent-accommodating part of the stent-accommodating tube body 5. The stent-accommodating tube body 5 has the side hole disposed on the proximal side relative to the stent-accommodating part. A guide wire can be passed via the side hole and the proximal-side opening.

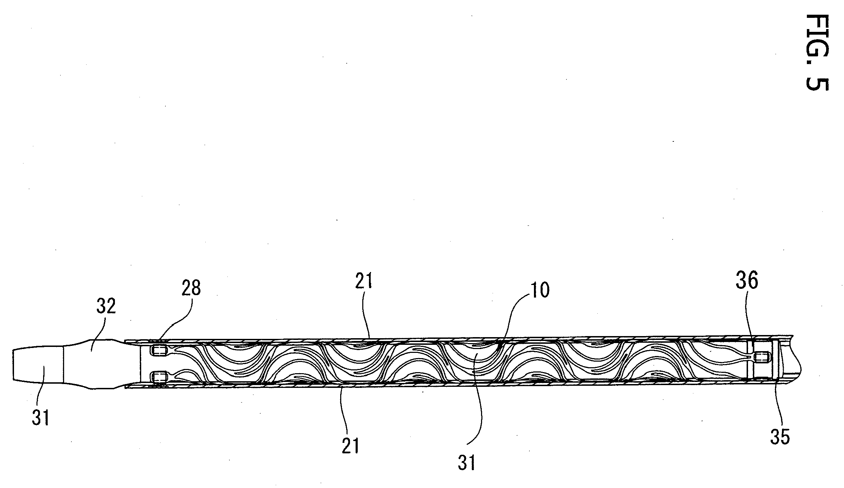

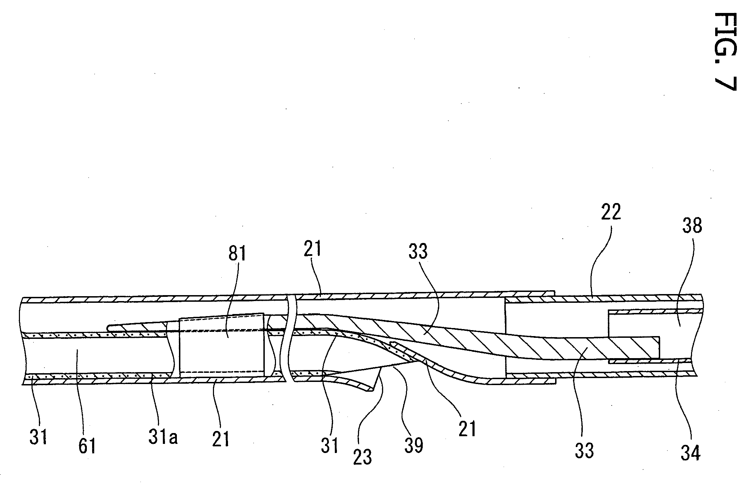

As shown in FIG. 5, the distal end of the distal-side tube 31 protrudes beyond the distal end of the stent-accommodating tube body 5 (the distal tube 21). In addition, the distal-side tube 31 is provided with a stopper 32 which inhibits movement of the stent-accommodating tube body 5 in the distal direction. As shown in FIG. 7, the proximal end portion of the distal-side tube 31 is curved, enters into the side hole 23 of the distal tube 21, and is disengageably engaged with the latter. The outside diameter of the distal-side tube 31 is preferably 0.2 to 2.0 mm. Besides, as shown in FIG. 5, a distal end portion of the distal-side stopper 32 is preferably decreased in diameter toward the distal end. The outside diameter at a greatest-diameter portion of the stopper 32 is preferably 0.5 to 4.0 mm. In addition, it is preferable that a proximal end portion of the stopper 32 is also decreased in diameter toward the proximal end, as shown in FIG. 5. Besides, the distal-side tube 31 has the guide wire lumen 61 extending from the distal end to the proximal end thereof. The position of a proximal opening 39 of the guide wire lumen 61 is preferably located at a position deviated by 10 to 400 mm, particularly 50 to 350 mm, to the proximal side from the distal end of the distal-side tube 31. In addition, the position of the proximal opening 39 is preferably deviated by about 50 to 250 mm to the proximal side from the proximal end of the stent 10 to be disposed (in other words, the proximal end of the stent-accommodating part).

Besides, the stent delivery system 1 according to the present invention is provided with the proximal-side tube 34 penetrating the stent-accommodating tube body 5, and with the stent-holding function by which the stent 10 is releasably held (preferably, the proximal end portion of the stent is releasably held) and by which it is ensures that, after partial exposure of the stent 10 from the stent-accommodating tube body 5, the stent 10 can be re-accommodated into the stent-accommodating tube body 5 by moving the stent-accommodating tube body 5 forward.

In the stent delivery system 1 in this embodiment, as the stent-holding function, the following configuration is provided. The inner tube body 3 includes: a distal-side contact section 36 which is located inside the proximal end portion of the stent 10 at such a position as not to enter the side-wall openings of the stent 10; and a proximal-side contact section 35 which is provided at a position rearward of the proximal end of the stent 10 and in proximity to the distal-side contact section 36 and which can be brought into contact with the proximal end of the stent 10. The stent 10 is provided with a proximal-side inwardly projecting section 17a capable of making contact with the distal-side contact section 36 of the inner tube body 3. Furthermore, the stent 10 is so disposed that the proximal-side inwardly projecting section 17a is located between the distal-side contact section 36 and the proximal-side contact section 35 of the inner tube body 3. This configuration ensures that, after partial exposure of the stent 10 from the stent-accommodating tube body 5, the stent 10 can be re-accommodated into the stent-accommodating tube body 5 by forward movement of the stent-accommodating tube body 5.

In addition, the stent 10 to be used in this embodiment is a so-called self-expandable stent which has the multiplicity of openings in the side surface thereof and which can be restored into its pre-compression shape by expanding outward at the time of indwelling in a living body. Further, the stent 10 used here has the distal end portion oriented toward the distal end of the stent-accommodating tube body 5 and the proximal end portion oriented toward the proximal end of the stent-accommodating tube body 5. Further, the stent 10 does not substantially have any bent free end at least oriented toward the proximal end, other than the proximal end portion. In addition, after the distal end portion of the stent 10 is exposed from the stent-accommodating tube body 5, the exposed distal end portion can be re-accommodated into the stent-accommodating tube body 5 by moving the stent-accommodating tube body 5 in the distal direction.

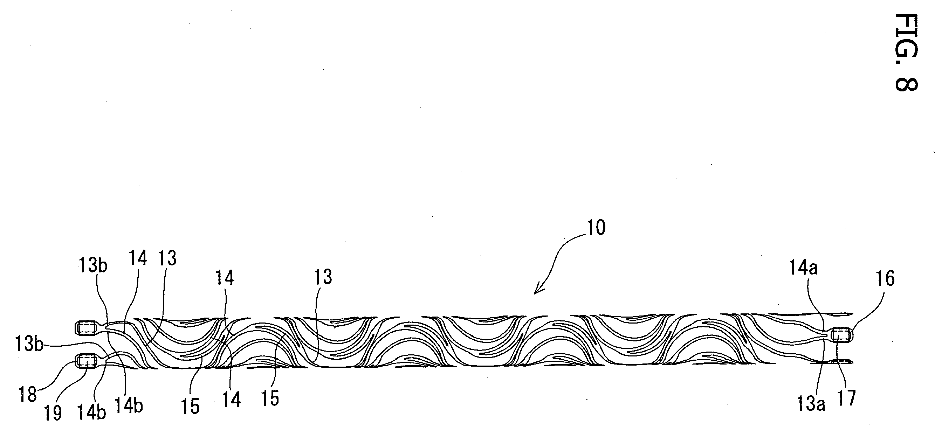

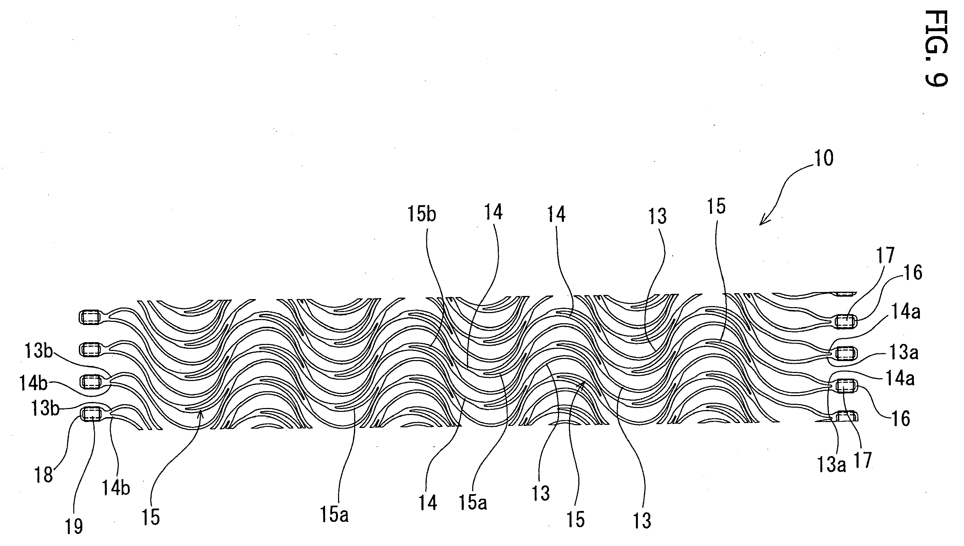

The stent to be used may be one in which an end portion of each filamentous component is connected to another filamentous component and which, therefore, does not have any free end. In addition, the stent to be used may be one as shown in FIGS. 8 and 9. FIG. 8 is a front view of an example of an in-vivo indwelling stent for use in the stent delivery system according to the present invention. FIG. 9 is a development view of the in-vivo indwelling stent of FIG. 8.

The stent 10 includes wavy struts 13, 14 extending in the axial direction from one end to the other end of the stent and arranged in plurality along a circumferential direction of the stent, and one or more link struts 15 interconnecting adjacent ones of the wavy struts and extending over a predetermined length along the axial direction. Furthermore, ends of the wavy struts 13, 14 are connected to ends of the adjacent wavy struts. In addition, the stent 10 has the multiplicity of openings formed between the struts.

Particularly, the stent 10 shown in FIGS. 8 and 9 includes: first wavy struts 13 extending in the axial direction from one end to the other end of the stent 10 and arranged in plurality along the circumferential direction of the stent; second wavy struts 14 each located between the first wavy struts 13, extending in the axial direction from one end to the other end of the stent, and arranged in plurality along the circumferential direction of the stent; and one or more link struts 15 each interlinking an adjacent pair of a first wavy strut 13 and a second wavy strut 14, and extending over the predetermined length in the axial direction. In addition, vertexes of the second wavy strut 14 are shifted by a predetermined length along the axial direction of the stent from vertexes of the first wavy strut 13 proximate thereto in the circumferential direction of the stent 10 and curved to the same direction. Besides, end portions 13a, 13b of the first wavy strut 13 are coupled to end portions 14a, 14b of the second wavy strut proximate thereto.

The stent 10 in this embodiment is a so-called self-expandable stent which is formed in a roughly cylindrical shape, is compressed toward the center axis at the time of insertion into a living body, and is restored into its pre-compression shape by expanding outward at the time of indwelling in the living body.

The first wavy struts 13 extend in the axial direction substantially in parallel to the center axis of the stent. In addition, the first wavy struts 13 are arranged in plurality along the circumferential direction of the stent. The number of the first wavy struts 13 is preferably three or more, particularly three to eight. Further, the plurality of first wavy struts 13 are preferably arranged at roughly regular angular intervals around the center axis of the stent.

The second wavy struts 14 also extend in the axial direction substantially in parallel to the center axis of the stent. In addition, the second wavy struts 14 are arranged in plurality along the circumferential direction of the stent, and are each disposed between the first wavy struts. The number of the second wavy struts 14 is preferably three or more, particularly three to eight. Further, the plurality of second wavy struts 14 are preferably arranged at roughly regular angular intervals around the center axis of the stent. Besides, the number of the second wavy struts 14 is preferably the same as the number of the first wavy struts 13.

In addition, the stent 10 has the one or more link struts 15 each of which interconnects an adjacent pair of the first wavy strut 13 and the second wavy strut 14 and which extend over the predetermined length in the axial direction. Particularly, in the stent 10 in this embodiment, the link strut 15 has one end in the vicinity of an inflection point of the wavy strut on one side, has the other end in a region ranging from the vicinity of a vertex of the adjacent wavy strut on the other side to a position a little beyond the vertex, extends in the axial direction, and is curved to the same direction as the vertex of the wavy strut on the other side. Besides, as shown in FIG. 9, the link strut 15 is composed of first link struts 15a which are curved and have vertexes directed toward one side in the circumferential direction of the stent 10 and second link struts 15b which are curved and have vertexes directed toward the other side in the circumferential direction of the stent 10. In addition, the link strut 15 is curved in an arcuate shape, and has a radius approximately equal to that of an arc of a curved portion of the first wavy strut 13 or the second wavy strut 14 which is proximate thereto in the circumferential direction of the stent 10.

Besides, the stent 10 in this embodiment has coupling sections 16, 18 by which an end portion of every one of the first wavy struts 13 is coupled to an end portion of either of the proximate second wavy struts. Specifically, one-end-side end portion 13a of the first wavy strut 13 of the stent 10 is coupled to one-end-side end portion 14a of one of the second wavy struts 14 proximate to the first wavy strut 13 (specifically, the second wavy strut 14 which is proximate to, and located on the circumferential-directionally other side of, the first wavy strut 13) by the coupling section 16. In addition, other-end-side end portion 13b of the first wavy strut 13 is coupled to other-end-side end portion 14b of one of the second wavy struts 14 proximate to the first wavy strut 13 (specifically, the second wavy strut 14 proximate to, and located on the circumferential-directionally one side of, the first wavy strut 13) by the coupling section 18. In other words, at the coupling section 16 on one end side and at the coupling section 18 on the other end side, the combinations of the first wavy strut 13 and the second wavy strut 14 coupled to each other are different (are shifted by one at a time).

In addition, as shown in FIG. 6, the stent 10 has the proximal-side inwardly projecting section 17a capable of making contact with the distal-side contact section 36 of the inner tube body 3. The stent 10 is so disposed that the proximal-side inwardly projecting section 17a is located between the distal-side contact section 36 and the proximal-side contact section 35 of the inner tube body 3. The proximal-side inwardly projecting section 17a is preferably composed of a radiopaque marker (radiopaque marker) 17 mounted to the proximal end portion (coupling section) 16 of the stent 10. Besides, as shown in FIG. 6, the proximal-side inwardly projecting section 17a of the stent 10 does not make contact with an outer surface of the distal-side tube 31 of the inner tube body 3. Incidentally, the proximal-side inwardly projecting section of the stent 10 may be composed of a thick wall section formed at the proximal end portion (coupling section) of the stent. The height of projection of the proximal-side inwardly projecting section is preferably 0.05 to 0.2 mm. In addition, the difference in height between the proximal-side inwardly projecting section of the stent and other non-projecting section is preferably 0.01 to 0.1 mm.

Furthermore, as shown in FIG. 6, the stent 10 in this embodiment may have a distal-side inwardly projecting section 19a at the distal end portion of the stent 10. The distal-side inwardly projecting section 19a is preferably composed of a radiopaque marker 19 mounted to the distal end portion (coupling section) 18 of the stent. Incidentally, the distal-side inwardly projecting section of the stent may be composed of a thick wall section formed at the distal end portion (coupling section) of the stent.

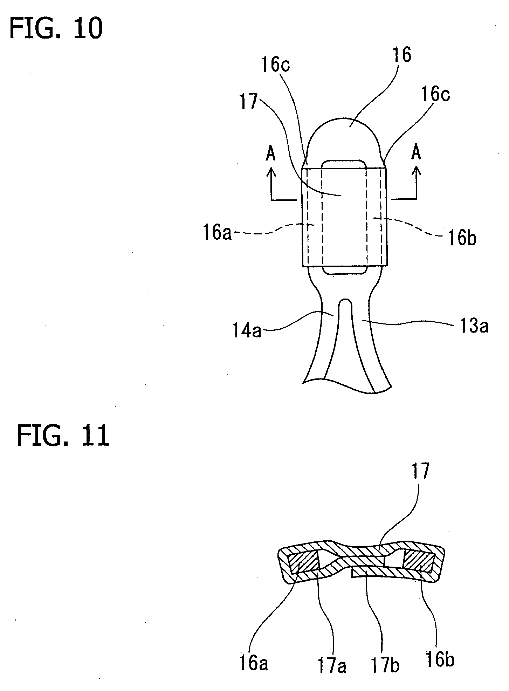

In the stent in this embodiment, the radiopaque marker 17 is attached to the coupling section 16. In this embodiment, the coupling section 16 has an opening, and has two frame sections 16a and 16b which extend in parallel in the direction toward the proximal end (end portion of a connecting section) of the stent, with a predetermined interval therebetween. The radiopaque marker 17 is attached to the coupling section 16. In this embodiment, the coupling section 16 has an opening, and has two frame sections 16a and 16b which extend in parallel in the direction toward the proximal end (end portion of a connecting section) of the stent, with a predetermined interval therebetween. The radiopaque marker 17 envelops substantially the whole part of the two frame sections 16a, 16b. In addition, the proximal-side inwardly projecting section 17a of the stent 10 is composed of a portion of the radiopaque marker 17 on the side of the inner surface of the stent. Further, in the stent in this embodiment, as shown in FIGS. 10 and 11, the proximal-side inwardly projecting section 17a of the stent 10 is formed of a portion of a sheet-formed member wound around the opening of the proximal end portion (coupling section) 16 of the stent 10 on the side of the inner surface of the stent. Furthermore, in the stent in this embodiment, the sheet-formed member has an inner overlapping section 17b projecting to the side of the inner surface of the stent 10, to form a portion which projects more than other portions.

The radiopaque marker 17 forming the proximal-side inwardly projecting section preferably has a predetermined thickness (line diameter). In addition, in

Incidentally, the proximal end portion (coupling section) of the stent may not have any independent opening as shown in FIGS. 10 and 11. For instance, a configuration may be adopted in which the proximal end of the end portion 14a of the strut is continuous with an end portion of the frame section 16a while the proximal end of the end portion 13a of the strut is continuous with the frame section 16b, the opening is opened at an end portion thereof, and the opening communicates with a space between the two struts.

Further, the proximal end portion (coupling section) of the stent may be one that does not have the above-mentioned opening at all. In this type of stent, a coupling section is a plate-formed section having a predetermined area and being a little curved, and a radiopaque marker is so attached as to cover a face and a back face of the plate-formed section.

Besides, the proximal end portion of the stent is preferably provided with a lock section 16c for restraining movement in the proximal direction of the radiopaque marker 17 which forms the proximal-side inwardly projecting section of the stent 10. Particularly, it is preferable that two such lock sections 16c are disposed opposite to each other, as shown in FIG. 10. With such lock sections provided, it is ensured that, at the time of re-accommodation of the stent 10 into the stent-accommodating tube body 5, the radiopaque marker 17 can be prevented from being moved relative to or disengaged from the stent when the radiopaque marker 17 is pressed toward the proximal end of the stent by the distal-side contact section 36 of the inner tube body 3. In addition, the proximal end portion 16 of the stent is protruding in the proximal direction beyond the radiopaque marker 17. Therefore, at the time of releasing the stent, the proximal-side contact section 35 of the inner tube body 3 makes contact with the proximal end of the proximal end portion 16 of the stent 10, and the proximal-side contact section 35 of the inner tube body 3 does not make contact with the radiopaque marker 17. Accordingly, the radiopaque marker 17 would not be moved relative to or disengaged from the stent.

Incidentally, in the whole of the above-described embodiments, as the radiopaque marker, the above-mentioned sheet-formed member is preferably used, but one formed by winding a wire-formed member around the proximal end portion (coupling section) of the stent may also be used. Also in this case, further, it is preferable to provide an inner overlapping section which projects to the side of the inner surface of the stent. The material to be preferably used for forming the above-mentioned radiopaque marker is one element (simple substance) or two or more elements (alloy) selected from the element group consisting of iridium, platinum, gold, rhenium, tungsten, palladium, rhodium, tantalum, silver, ruthenium, and hafnium.

Besides, fixation of the radiopaque marker can be carried out by any of welding, soldering, adhesion, fusing, and diffusion.

A material forming the stent 10 is preferably a superelastic metal. As the superelastic metal, a superelastic alloy is preferably used. The superelastic alloy here means a metal which is commonly called a shape-memory alloy and which exhibits superelasticity at least at a living body temperature (around 37°C). Particularly preferable examples are such superelastic alloys as Ti-Ni alloy containing 49 to 53 at% of Ni, Cu-Zn alloy containing 38.5 to 41.5 wt% of Zn, Cu-Zn-X alloys (X = Be, Si, Sn, Al, or Ga) containing 1 to 10 wt% of X, and Ni-Al alloy containing 36 to 38 at% of Al. Especially preferred is the above-mentioned Ti-Ni alloy. In addition, mechanical properties can be appropriately modified by replacing part of the Ti-Ni alloy with 0.01 to 10.0 wt% of X to obtain Ti-Ni-X alloys (X = Co, Fe, Mn, Cr, V, Al, Nb, W, B or the like), or by replacing part of the Ti-Ni alloy with 0.01 to 30.0 at% of X to obtain Ti-Ni-X alloys (X = Cu, Pb, or Zr), or by selecting cold working ratio or/and final heat treatment conditions. Besides, the above-mentioned Ti-Ni-X alloys may be used and cold working ratio and/or final heat treatment conditions may be selected, whereby mechanical properties can be appropriately changed. The buckling strength (yield stress when loaded) of the superelastic alloy to be used is 5 to 200 kg/mm² (22°C), more preferably 8 to 150 kg/mm², and the restoring stress (yield stress when unloaded) of the superelastic alloy is 3 to 180 kg/mm² (22°C), more preferably 5 to 130 kg/mm². The superelasticity here means a property such that even if the material is subjected to deformation (bending, stretching, or compression) into a range for ordinary metals to be plastically deformed at use temperature, the material is restored substantially into its pre-compression shape without heating after release from the deformation.

In addition, the diameter of the stent when compressed is preferably 0.5 to 1.8 mm, particularly preferably 0.6 to 1.4 mm. Besides, the length of the stent when not compressed is preferably 5 to 200 mm, particularly preferably 8.0 to 100.0 mm. In addition, the diameter of the stent when not compressed is preferably 1.5 to 6.0 mm, particularly preferably 2.0 to 5.0 mm. Further, the material thickness of the stent is preferably 0.05 to 0.15 mm, particularly preferably 0.05 to 0.40 mm. The width of the wavy struts is preferably 0.01 to 1.00 mm, particularly preferably 0.05 to 0.2 mm. Surfaces of the wavy struts have been preferably smoothened, more preferably been smoothened by electropolishing. In addition, the strength in radial direction of the stent is preferably 0.1 to 30.0 N/cm, particularly preferably 0.5 to 5.0 N/cm.

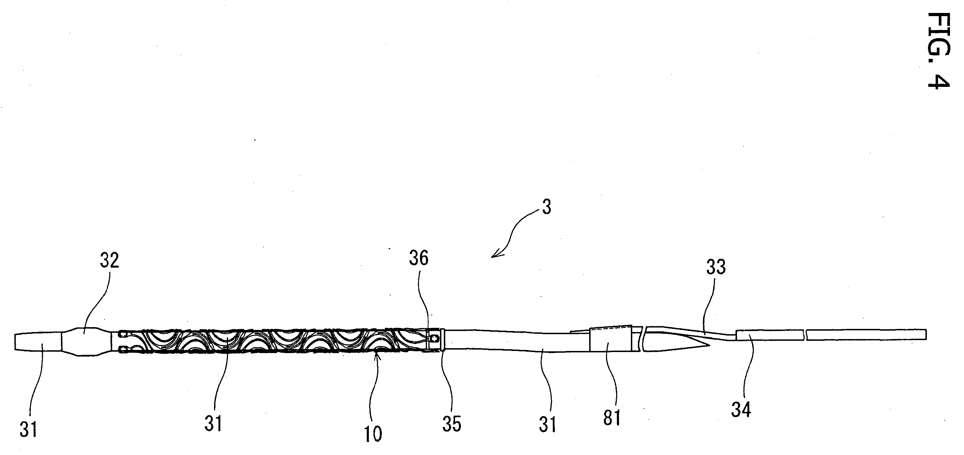

Besides, as shown in FIGS. 4, 5, and 6 (particularly, in FIG. 6), the inner tube body 3 has: the distal-side contact section 36 which is located in the proximal end portion of the stent 10 and which does not enter the side-wall openings of the stent 10; and the proximal-side contact section 35 which is disposed at a position rearward of the proximal end of the stent 10 and proximate to the distal-side contact section 36 and which is able to make contact with the proximal end of the stent 10. In addition, in this embodiment, as shown in FIGS. 4, 5, and 6 (especially, in FIG. 6), the distal-side contact section 36 is a distal-side projecting section projecting from the outer surface of the distal-side tube 31; like the distal-side contact section 36, the proximal-side contact section 35 is also a proximal-side projecting section projecting from the outer surface of the distal-side tube 31.

Besides, the proximal-side inwardly projecting section 17a of the stent 10 mentioned above is capable of making contact with the distal-side contact section 36 of the inner tube body 3. In addition, as shown in FIG. 6, the proximal-side inwardly projecting section 17a of the stent 10 is located between the distal-side contact section 36 and the proximal-side contact section 35 of the inner tube body 3.

Besides, as shown in FIGS. 5 and 6, the stent delivery system 1 in this embodiment has the distal-side contact section 36 at a position deviated by a predetermined distance toward the proximal end from the distal end of the distal-side tube 31. The distal-side contact section 36 is disposed at a position which is inside the proximal end portion of the stent 10 and which is deviated a little toward the distal end relative to the proximal end of the stent 10. In addition, the proximal-side contact section 35 is disposed at a position which is deviated a little toward the proximal end relative to the distal-side contact section 36. The proximal-side contact section 35 is disposed in the vicinity of and rearward (proximal side) of the proximal end of the stent 10. The proximal-side inwardly projecting section 17a of the stent 10 is located between the distal-side contact section 36 and the proximal-side contact section 35 of the inner tube body 3. Therefore, the distance between the distal-side contact section 36 and the proximal-side contact section 35 is slightly longer than an axial length of the proximal-side inwardly projecting section 17a of the stent 10. The distance between the distal-side contact section 36 and the proximal-side contact section 35 is preferably longer than the axial length of the proximal-side inwardly projecting section 17a of the stent 10 by 0.02 to 1.0 mm, particularly preferably by 0.05 to 0.3 mm.

In addition, the distal-side contact section 36 does not enter the side-wall openings of the stent 10. Besides, the distal-side contact section 36 is preferably an annular projecting section disposed continuously over the outer circumference of the distal-side tube 31. The annular projecting section is formed, for example, by attaching a tubular member to the outer circumference of the distal-side tube. With such an annular projecting section, assured contact thereof with the proximal-side inwardly projecting section 17a of the stent 10 is realized. In addition, the distal-side contact section 36 is preferably a section which substantially does not make contact with the inner surface of the stent 10. This prevents the distal-side contact section 36 from constituting an obstacle at the time of releasing the stent. The distal-side contact section 36 has such a height that it can make contact with the proximal-side inwardly projecting section 17a of the stent 10. The height of the distal-side contact section 36 is preferably 0.06 to 0.11 mm, particularly preferably 0.08 to 0.11 mm. Besides, an axial length of the distal-side contact section 36 is preferably 0.1 to 3.0 mm, particularly preferably 0.3 to 2.0 mm.

Incidentally, while the distal-side contact section 36 is preferably the annular projecting section disposed continuously over the outer circumference of the distal-side tube 31, it may be composed of a plurality of discontinuous ribs arranged in an annular pattern.

In addition, the proximal-side contact section 35 is preferably an annular projecting section disposed continuously over the outer circumference of the distal-side tube 31. The annular projecting section is formed, for example, by attaching a tubular member to the outer circumference of the distal-side tube. Besides, the proximal-side contact section 35 is preferably a section which does not make contact with the inner surface of the stent-accommodating tube body 5. This prevents the proximal-side contact section 35 from obstructing an operation at the time of releasing the stent. The proximal-side contact section 35 has such a height that it can make contact with the proximal end of the stent 10. The height of the proximal-side contact section 35 is preferably 0.08 to 0.18 mm, particularly preferably 0.1 to 0.16 mm. In addition, an axial length of the proximal-side contact section 35 is preferably 0.1 to 3.0 mm, particularly preferably 0.3 to 2.0 mm. Besides, the distance between an outer surface of the proximal-side contact section 35 and an inner surface of the stent-accommodating tube body 5 is preferably 0.01 to 0.04 mm. In addition, it is preferable that the proximal-side contact section 35 is greater in height than the distal-side contact section 36, and that the difference in height between these sections is 0.02 to 0.1 mm.

Further, the distal-side contact section 36 and the proximal-side contact section 35 are preferably formed from a radiopaque material. As the radiopaque material, the materials for forming the radiopaque marker as above-mentioned can be used suitably. Particularly, it is preferable for these contact sections to be each formed by attaching a tubular member formed of the radiopaque material. Furthermore, it is preferable that the distal-side contact section 36 and the proximal-side contact section 35 are formed from the radiopaque material, and that they are different from each other in axial length. This promises easy discrimination between them. While which one of them is longer does not matter, the difference between them in axial length is preferably 0.3 to 1.0 mm.

Besides, in the stent delivery system 1 in this embodiment, the inner tube body 3 (specifically, the distal-side tube 31) has the opening 39 which communicates with the guide wire lumen on the proximal side relative to the stent-accommodating part of the

Besides, in the stent delivery system 1 in this embodiment, the inner tube body 3 (specifically, the distal-side tube 31) has the opening 39 which communicates with the guide wire lumen on the proximal side relative to the stent-accommodating part of the stent-accommodating tube body 5.

In addition, the distal-side tube 31 may have a reinforcement layer 31a at least along a portion located on the proximal side relative to the proximal end of the stent, as shown in FIG. 7. The reinforcement layer 31a is preferably disposed over the whole part of the distal-side tube 31. Incidentally, a structure may be adopted in which the reinforcement layer 31a is not disposed at a distal portion of the distal-side tube 31. The reinforcement layer 31a is preferably a network-formed reinforcement layer. The network-formed reinforcement layer is preferably formed from braids. The braids can be formed, for example, from metallic wire of stainless steel, elastic metal, superelastic alloy, shape-memory alloy or the like having a wire diameter of 0.01 to 0.2 mm, preferably 0.03 to 0.1 mm. Besides, the braids may be formed from synthetic fiber such as polyamide fiber, polyester fiber, and polypropylene fiber. connector 46 fixed to the proximal end of the proximal-side tube 34.

The proximal-side tube 34 of the inner tube body is a tube having the penetrating internal lumen which has its distal end opening in the stent-accommodating tube body 5 (specifically, in a distal end portion of the proximal tube 22 of the stent-accommodating tube body 5) and has its proximal end opening in the connector 46.

In addition, as shown in FIG. 7, the inner tube body 3 has the connection member which interconnects the distal-side tube 31 and the proximal-side tube 34. In this embodiment, the connection member is composed of the wire-formed member 33 and a heat-shrinkable tube 81. A proximal end portion of the wire-formed member 33 enters the distal end portion of the proximal-side tube 34, and is fixed there. Besides, a distal end portion of the wire-formed member 33 is fixed to a side surface of the distal-side tube 31 by the heat-shrinkable tube 81. Further, in this embodiment, the wire-formed member 33 has a smaller diameter section on the distal side of a portion fixed to the distal-side tube 31.

The length of the inner tube body 3 is preferably 400 to 2,500 mm, particularly preferably 400 to 2,200 mm. In addition, the outside diameter of the proximal-side tube 34 is preferably 0.3 to 3.0 mm, particularly preferably 0.5 to 1.0 mm. Besides, the inside diameter of the proximal-side tube 34 is preferably 0.1 to 2.5 mm, particularly preferably 0.2 to 2.0 mm. The length of the distal-side tube 31 is preferably 10 to 400 mm, particularly preferably 50 to 350 mm. The outside diameter of the distal-side tube 31 is preferably 0.2 to 2.0 mm, particularly preferably 0.4 to 1.7 mm. In addition, the inside diameter of the lumen 61 is preferably 0.1 to 1.8 mm, particularly preferably 0.3 to 1.0 mm.

A material for forming the inner tube body (the distal-side tube 31 and the proximal-side tube 34) is preferably a material which has hardness and a certain degree of flexibility. Examples of the material which can be used suitably include stainless steel, superelastic metal, polyethylene, polypropylene, nylon, polyethylene terephthalate, fluoro-polymers such as ETFE, PEEK (polyether ether ketone), and polyimide. Incidentally, an outer surface of the inner tube body 3 may be coated with a biocompatible material, particularly an antithrombogenic material. Examples of the antithrombogenic material which can be used suitably include polyhydroxyethyl methacrylate, and hydroxyethyl methacrylate-styrene copolymer (for example, HEMA-St-HEMA block copolymer).

Furthermore, of the inner tube body 3, a part which may protrude beyond the stent-accommodating tube body 5 preferably has a lubricating outer surface. For this purpose, the outer surface may be coated with a hydrophilic polymer such as poly (2-hydroxyethyl methacrylate), polyhydroxyethyl acrylate, hydroxypropyl cellulose, methyl vinyl ether-maleic anhydride copolymer, polyethylene glycol, polyacrylamide, polyvinylpyrrolidone, and dimethylacrylamide-glycidyl methacrylate copolymer, or the hydrophilic polymer may be fixed to the outer surface. In addition, the whole outer surface of the inner tube body 3 may be coated with the just-mentioned hydrophilic polymer, or the hydrophilic polymer may be fixed to the outer surface. Further, an inner surface of the inner tube body 3 may also be coated with the just-mentioned hydrophilic polymer, or the hydrophilic polymer may be fixed to the inner surface, for enhancing the sliding properties of the inner surface for a guide wire.

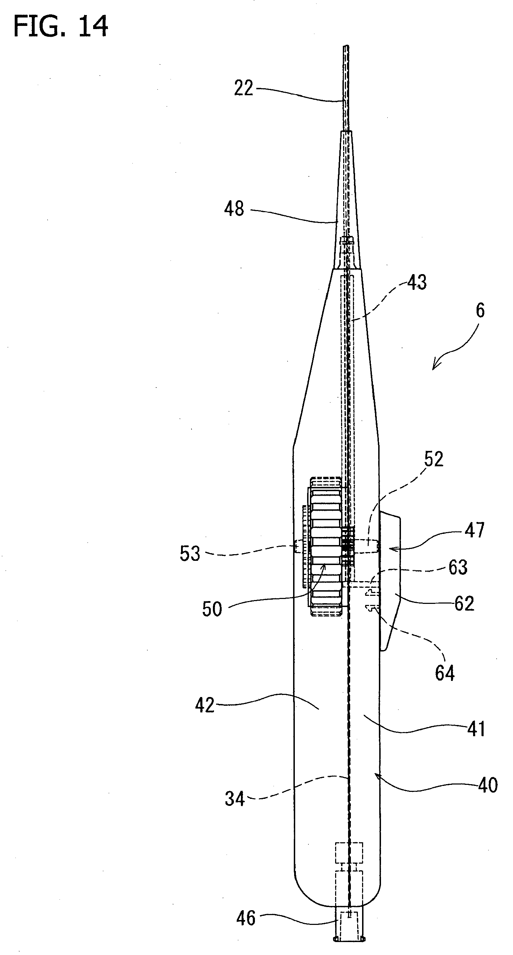

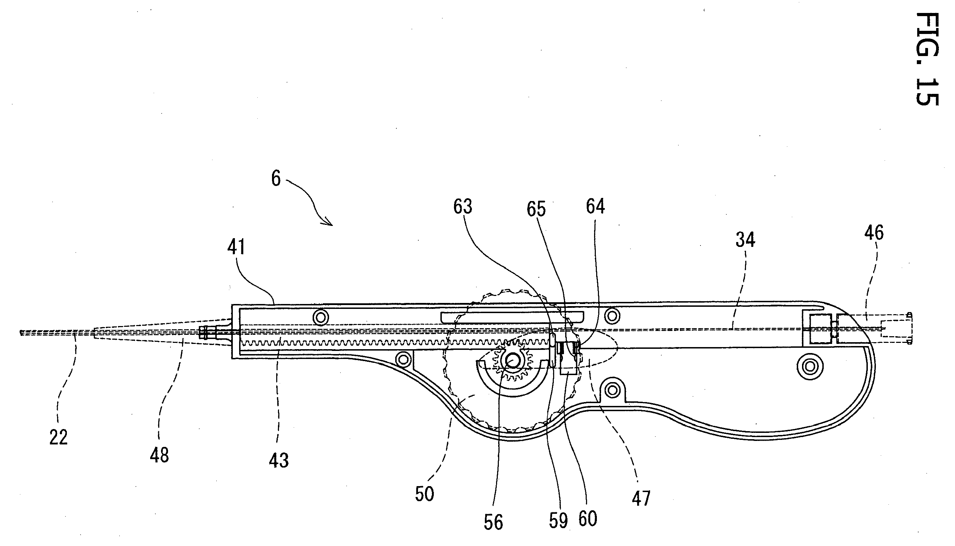

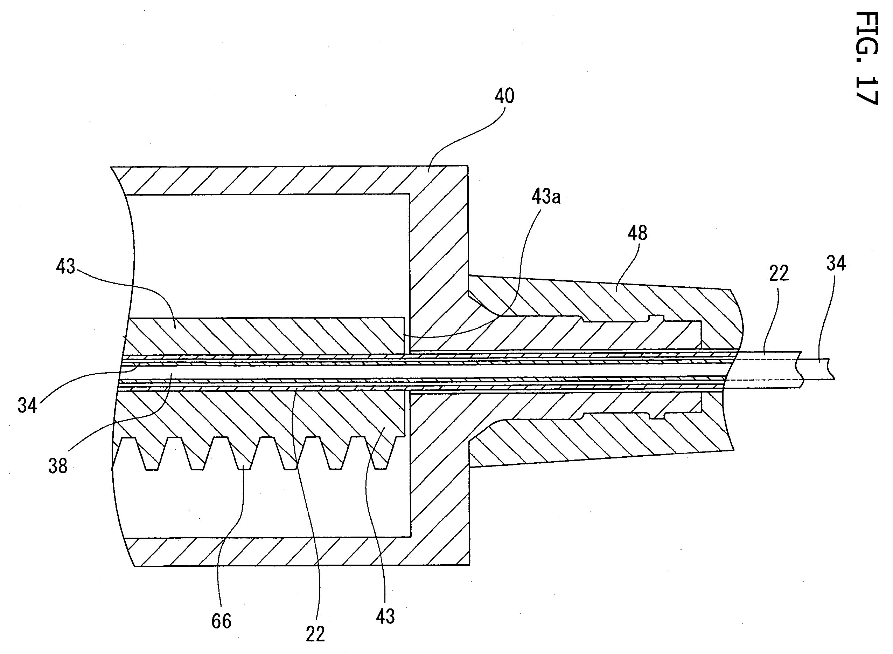

In addition, the proximal-side tube 34 penetrates the stent-accommodating tube body and protrudes beyond the proximal opening of the stent-accommodating tube body 5 (the proximal tube 22). As shown in FIG. 1 and 13 to 19, the connector 46 is firmly attached to the proximal end portion of the proximal-side tube 34.

Besides, in the stent delivery system 1, a liquid-injecting device can be connected to the connector 46. A liquid injected by the liquid-injecting device thus connected passes through the lumen 38 inside the proximal-side tube 34, and flows out in a distal-side portion of the stent delivery system (the stent-accommodating tube body), whereby the inside of the stent delivery system (the stent-accommodating tube body) can be flushed. Furthermore, the liquid can be ejected via the distal end of the stent delivery system (the stent-accommodating tube body).

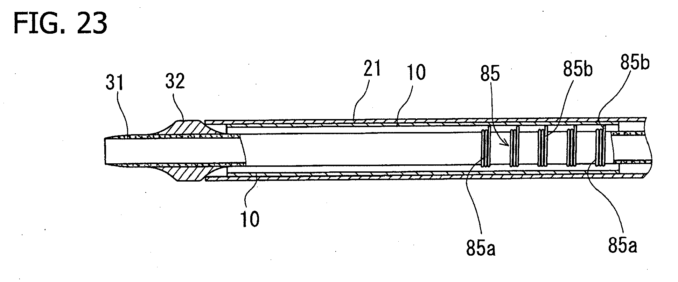

In addition, the stent-holding function for enabling the stent 10 possessed by the stent delivery system 1 according to the present invention to be re-accommodated into the stent-accommodating tube body 5 by forward movement of the stent-accommodating tube body 5 after partial exposure of the stent 10 from the stent-accommodating tube body 5 is not restricted to the one configured as above-described. For instance, the stent-holding function may be one as shown in FIG. 23.

In the configuration according to this embodiment, the inner tube body 3 has an elastic member 85 which is disposed at least on the outer surface of the inner tube body 3 located inside the proximal end portion of the stent and which press the stent 10 toward the stent-accommodating tube body 5. Besides, the stent 10 is gripped between the elastic member 85 and the stent-accommodating tube body 5, and is slidable relative to the stent-accommodating tube body 5. In addition, the stent 10 is substantially non-slidable relative to the elastic member 85.

The elastic member 85 is fixed onto the outer surface of the inner tube body 3 (specifically, the distal-side tube 31). Besides, as shown in FIG. 23, the elastic member 85 is a wire coil which has a fixation section 85a for fixation to the distal-side tube 31 and an elastic section 85b for pressing the stent 10. The fixation section 85a is formed by winding around the distal-side tube 31 a wire for constituting the wire coil. As shown in FIG. 23, the elastic section 85b is configured by a method in which the wire forming the fixation section 85a is spaced apart from the distal-side tube 31 while being enlarged in diameter. The elastic section 85b has such a size and such a spring elasticity as to be able to press the stent 10 accommodated in the stent-accommodating tube body 5. In addition, in this embodiment, at least the elastic section 85b of the elastic member 85 composed of the wire coil is preferably a little inclined toward the proximal end, as shown in FIG. 23. Incidentally, at least the elastic section 85b of the elastic member 85 composed of the wire coil may be a little inclined toward the distal end. Where the elastic section 85b of the elastic member 85 composed of the wire coil is thus inclined, better pressing of the stent 10 is ensured. Besides, in this embodiment, the elastic member 85 presses a part of the inner circumference of the stent 10, as shown in FIG. 23.

In addition, a plurality of such elastic members 85 is disposed. Particularly, in the stent delivery system 1 shown in FIG. 23, the plurality of elastic members 85 is disposed within the proximal end portion of the stent 10. Besides, the elastic members 85 are arranged at substantially regular intervals. Incidentally, the plurality of elastic members may be arranged so that the interval thereof decreases along the direction toward the proximal end of the stent.

Now, the operation unit 6 of the stent delivery system 1 according to the present invention will be described below.

The operation unit 6 has the moving mechanism for moving the stent-accommodating tube body 5. In this embodiment, the operation unit 6 includes: the housing 40; the shaft-like rack member 43 which is accommodated in the housing 40 and which is fixed to the proximal end of the stent-accommodating tube body 5 (specifically, the proximal tube 22); the operation rotary roller 50 having the working gear wheel 54 which engages with the teeth 66 of the rack member 43 and which is for moving the rack member 43 within the housing 40; and the connector 46 fixed to the proximal end portion of the proximal-side tube 34 which penetrates the stent-accommodating tube body 5 (specifically, the proximal tube 22) fixed to the rack member 43 and which protrudes beyond the proximal end of the stent-accommodating tube body 5.

The operation unit 6 in this embodiment has the moving mechanism for moving the shaft-like rack member 43 backward and forward, whereby the stent-accommodating tube body 5 fixed to the rack member 43 can be brought into withdrawal (exposure of the stent) and advance (accommodation of the stent) relative to the inner tube body 3.

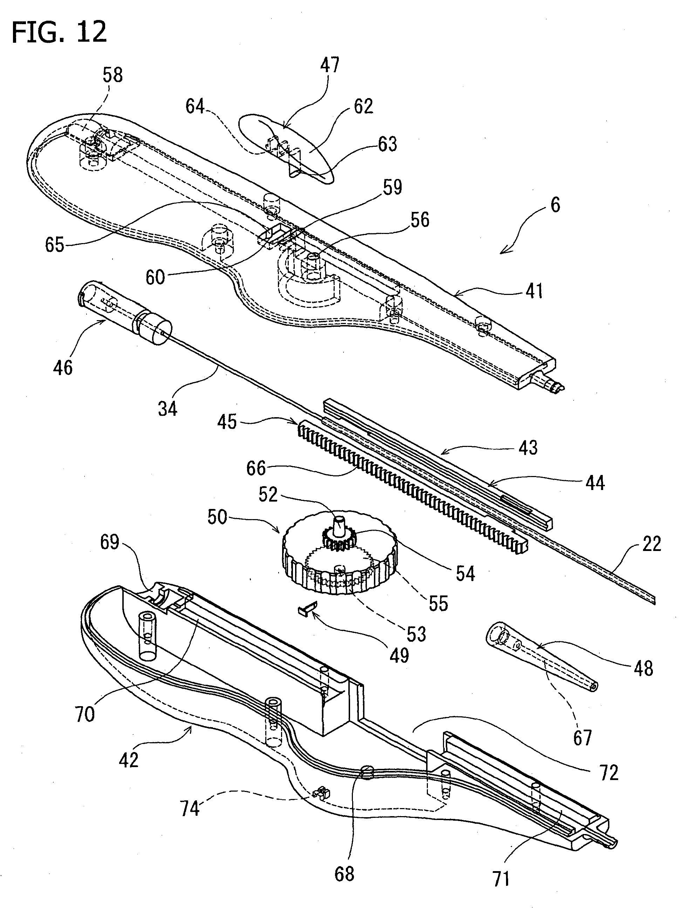

As shown in FIGS. 12 to 19, the operation unit 6 has the housing 40. The housing 40 is composed of a first housing 41 and a second housing 42. The housing 40 has a shape which is bent and rounded at a proximal end side and at a central portion, which enables easy gripping, and which enables easy operation of the roller in a condition where the housing 40 is gripped.

As shown in FIGS. 12 to 19, the housing 40 has: an opening 72 for permitting the operation rotary roller 50 to be partially protruded from an accommodating section; a bearing section 56 for accommodating one end 52 of a rotary shaft of the roller 50; and a bearing section 68 for accommodating the other end 53 of the rotary shaft of the roller 50.

In addition, as shown in FIGS. 12 to 19, the connector 46 in a tubular shape is fixed to the proximal end portion of the proximal-side tube 34 of the inner tube body 3, and the housing 40 has an accommodating section 58, 69 for the connector 46. Besides, the shaft-like rack member 43 is fixed to a proximal end portion of the proximal tube 22 of the stent-accommodating tube body 5, and the housing 40 has a rack member accommodating section 70, 71 in which the rack member 43 is accommodated in an axially movable manner. In addition, a distal member 48 to be fitted over the proximal tube 22 of the stent-accommodating tube body 5 so as to permit sliding of the proximal tube 22 is fixed to a distal end portion of the housing 40. The distal member 48 has therein with a passage 67 which the proximal tube 22 penetrates in a slidable manner.

As shown in FIG. 12, the rack member 43 is composed of a first rack member 44 and a second rack member 45, which are fixed to the proximal end portion of the proximal tube 22 of the stent-accommodating tube body 5 by clamping the proximal end portion of the proximal tube 22 between both of them. In addition, the rack member 43 has a shaft-like shape longer than the stent 10 by a predetermined length, and has the teeth 66 formed on a roller-side surface thereof (a surface facing to a lower side of the housing). The teeth 66 are formed on the whole part of a tooth-formed surface of the rack member 43.

As shown in FIGS. 12 to 19, the operation rotary roller 50 includes one end 52 of the rotary shaft disposed at a side surface on one side, the other end 53 of the rotary shaft disposed at a side surface on the other side, and the working gear wheel 54 which engages with the teeth 66 of the rack member 43 and which is for moving the rack member 43 within the housing 40. The working gear wheel 54 is smaller than the rotary roller in diameter. The outside diameter of the working gear wheel 54 is preferably 10 to 60 mm. In addition, the roller 50 has a gear-formed section 55 disposed for enabling intermittent rotation of the roller. The operation rotary roller 50 is partially exposed via the opening 72 of the housing 40, and the exposed part constitutes an operation part.

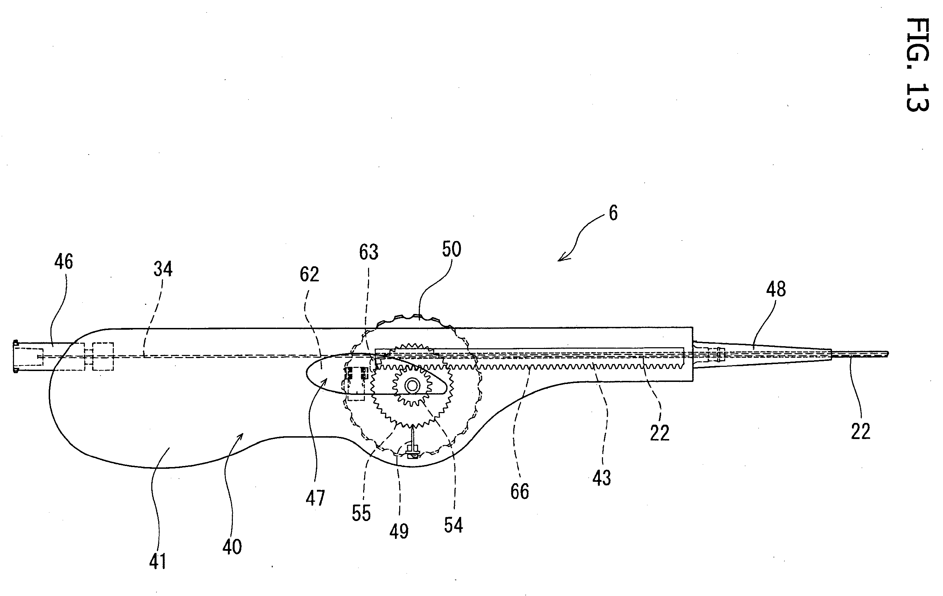

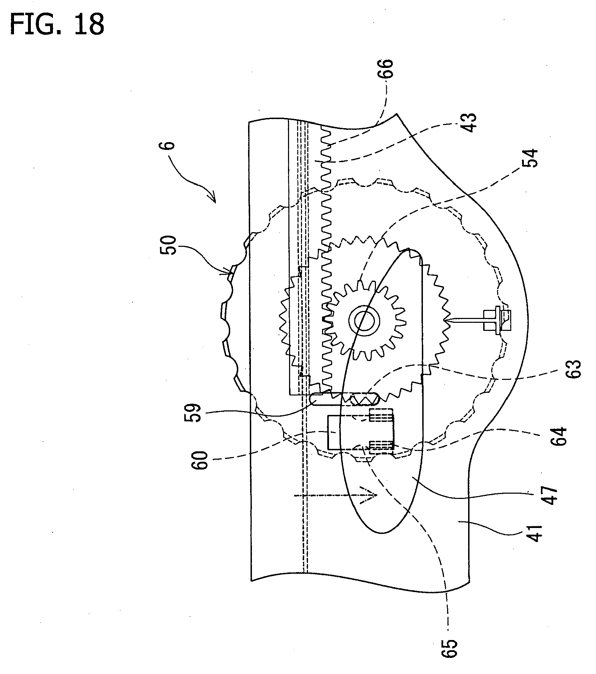

Besides, the operation unit 6 in this embodiment has a lock mechanism for releasably locking the rack member 43. The operation unit 6 has a lock lever 47. As shown in FIG. 12, the lock lever 47 includes a lock lever main body 62, a lock bar 63 protruding from the lock lever main body 62, and a mounting section 64 for mounting onto the housing. In addition, as shown in FIG. 12, the first housing 41 has a lock bar accommodating port 59 in which the lock bar 63 is slidably accommodated, and a mounting port 60 which slidably accommodates the mounting section 64 therein, engages with a distal end portion of the mounting section 64, and holds the lock lever 47. The mounting port 60 has a rib 65 for holding the lock lever 47 in a locked-state position and in an unlocked-state position. Besides, as shown in FIGS. 13 to 15, in a condition where the lock lever 47 is located on the side of the opening 72 of the housing 40, the lock bar 63 makes contact with a proximal end face of the rack member 43, thereby inhibiting the rack member 43 from moving backward (a direction toward the connector member 46; a stent-releasing direction). In addition, as shown in FIG. 18, with the lock lever 47 pressed down (pressed in a direction for coming away from the opening 72 of the housing 40), the lock lever mounting section 64 rides over the rib 65 formed on an inner surface of the mounting port 60, and slides downward (in a direction for coming away from the rack member 43) within the mounting port. The lock bar 63 also slides within the lock bar accommodating port 59 to move in the direction for coming away from the rack member 43. Consequently, the lock bar 63 comes out of contact with the proximal end face of the rack member 43, whereby locking is removed, and the roller 50 is permitted to be rotated. Besides, the unlocked state is held by the rib 65 formed on the inner surface of the mounting port 60.

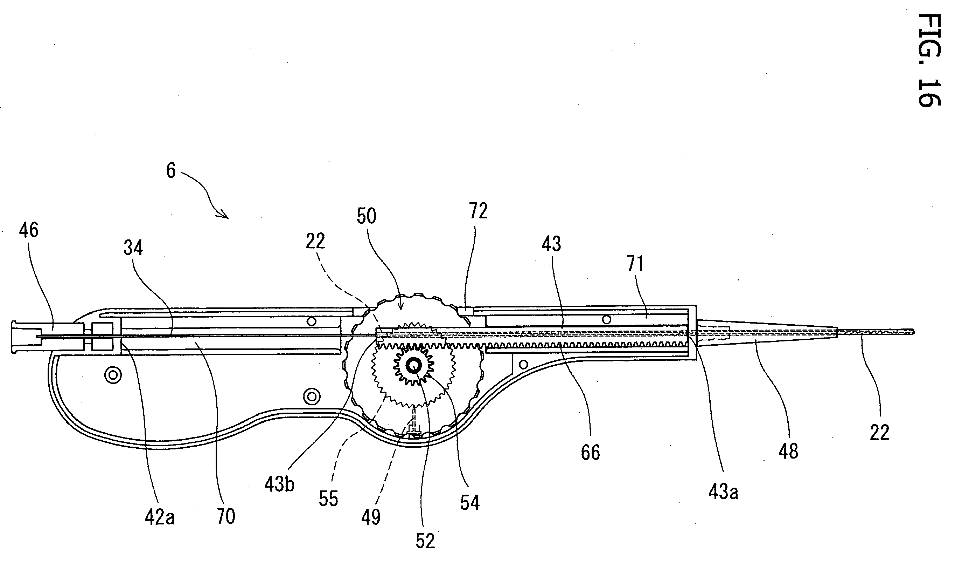

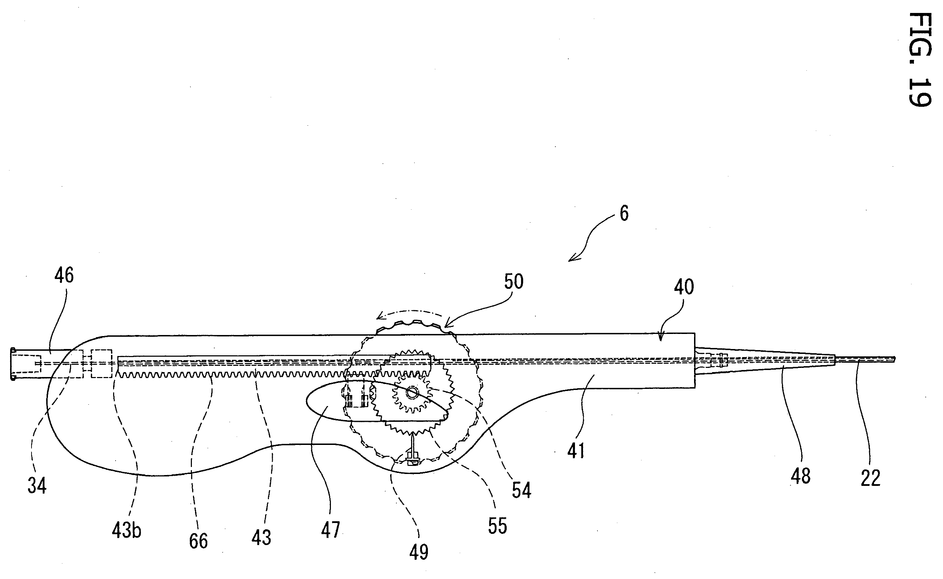

Further, the operation unit 6 in this embodiment has a movement-restraining section (rotation-restraining section) which, at the time of rotation of the operation rotary roller 50, makes contact with an end portion of the rack member to restrain movement of the rack member (in other words, rotation of the roller, or movement of the stent-accommodating tube body 5 relative to the inner tube body 3) in excess of a predetermined extent. Specifically, as shown in FIG. 16 which illustrates a condition where the first housing 41 has been detached from the operation unit 40, a distal end 43a of the rack member 43 is able to make contact with an inner surface of a distal end portion of the housing 40 (the second housing), and the rack member 43 is unable to advance further. Thus, the operation unit 6 in this embodiment has the movement-restraining section (rotation-restraining section) which, when the operation rotary roller 50 is rotated in the reverse direction to the predetermined direction (in an advancing direction of the stent-accommodating tube body 5; in a stent-accommodating direction), makes contact with the distal end 43a of the rack member 43 to thereby restrain further movement of the rack member (further rotation of the roller). Similarly, as shown in FIGS. 16 and 19, a proximal end 43b of the rack member 43 is able to make contact with an inner surface 42a of a proximal end portion of the housing 40 (the second housing), and the rack member 43 is unable to retreat further. Thus, the operation unit 6 in this embodiment has the movement-restraining section (rotation-restraining section) which, when the operation rotary roller 50 is rotated in the predetermined direction (in a retreating direction of the stent-accommodating tube body 5; in a stent-releasing direction), makes contact with the proximal end 43b of the rack member 43 to thereby restrain the rack member from moving (the roller from rotating) further (in excess of a predetermined extent).

Incidentally, in order to prevent the stent-accommodating tube body from being deformed or broken by an excessive force at the time of movement of the stent-accommodating tube body 5, a configuration may be adopted in which when a force in excess of a safety setpoint is exerted, engaging between the working gear wheel of the rotary roller and the teeth of the rack member is released, resulting in idling. Such an idling-generating mechanism can be formed, for example, by clearances in the bearing section 56 in which the one end 52 of the rotary shaft of the operation rotary roller 50 is accommodated and in the bearing section 68 in which the other end 53 of the rotary shaft of the roller 50 is accommodated. In addition, the above-mentioned safety setpoint is preferably set lower than the breaking strength of the stent-accommodating tube body.

Further, the operation unit 6 in this embodiment has a roller intermittent rotation mechanism which holds the operation rotary roller 50 to impart a rotation resistance and which permits intermittent rotation of the roller. In this embodiment, the roller intermittent rotation mechanism is composed of the gear-formed section 55 disposed on the operation rotary roller 50, and an elastically deformable pin 49 having a distal end portion entering a recess of the gear-formed section 55. The elastically deformable pin 49 has a body part extending in a direction toward the gear-formed section 55 and a proximal part disposed at a proximal end portion of the body part. The proximal part is fixed to a pin-fixing section 74 of the second housing 42. Incidentally, in this embodiment, the gear-formed section 55 is formed on a surface, different from a surface formed with the working gear wheel 54, of the roller 50. In addition, the roller 50 is pressed in a direction toward the opening 72 by the deformable pin 49, thereby being restrained from unprepared rotation. Besides, at the time of rotation of the roller 50, the pin 49 is deformed to permit rotation of the roller, and, when the distal end of the pin 49 enters the recess of the gear-formed section 55, a roller-holding state is attained, so that intermittent (stepwise) rotation of the roller is enabled. Furthermore, the pin 49 is preferably one that generates a sound upon restoration from a deformed state, during rotation of the roller. This enables an operator to recognize the rotation. Further, the pin 49 preferably generates rotational sounds discernible depending on the rotating direction of the roller. This makes it possible to confirm, by the sound, the direction in which the rotation is being carried out, in other words, whether the stent-accommodating tube body 5 is being moved forward or backward.

Now, operation of the stent delivery system according to the present invention will be described below, referring to FIGS. 18 to 22.

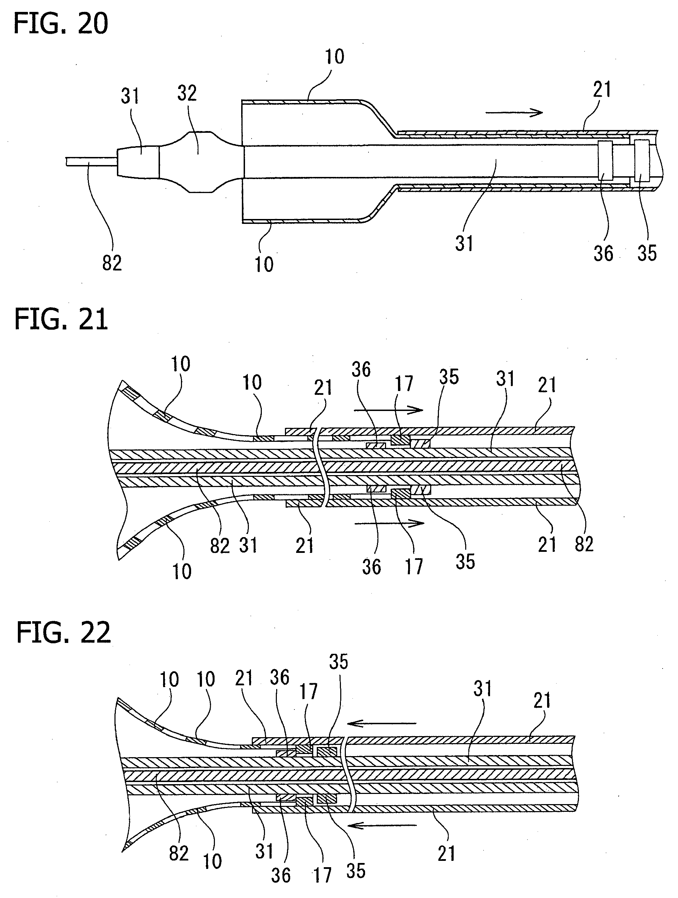

The stent delivery system 1 having a guide wire 82 passed through the distal-side tube 31 is inserted into a blood vessel to be treated, and the stent is brought to a target part. In this state, the whole part of the stent 10 is accommodated in the stent-accommodating tube body 5. Next, as shown in FIG. 18, the lock lever 47 is depressed, to unlock the rack member 43. Then, as shown in FIG. 19, the roller is rotated in the predetermined direction (in a direction of an arrow), whereby the stent-accommodating tube body 5 is moved backward relative to the inner tube body 3. This results in that the stent 10 is gradually exposed and permitted to expand, starting from the distal end side thereof, as shown in FIG. 20. In the stent delivery system in this embodiment, movement of the stent 10 in the proximal direction is restrained by the contact of the proximal end of the stent 10 with the proximal-side contact section 35 of the inner tube body 3 (the distal-side tube 31), as shown in FIG. 21; therefore, the stent 10 can be exposed. The stent 10 exposed from the stent-accommodating tube body 5 tends to expand by the self-expanding force, so as to be restored into its pre-compression shape. Thereafter, in a case where readjustment of the placing position of the stent 10 is needed, the roller is rotated in the reverse direction to the predetermined direction (the direction of the arrow). This results in that, as shown in FIG. 22, the stent-accommodating tube body 5 is moved in the distal direction, and part of or the whole part of the stent is re-accommodated into the stent-accommodating tube body 5 (the distal-side tube 31). In this embodiment, movement of the stent 10 in the distal direction is restrained by the contact of the proximal-side inwardly projecting section 17a of the stent 10 with the distal-side contact section 36 of the inner tube body 3 (the distal-side tube 31); therefore, the stent 10 can be accommodated.

Then, after the stent is re-placed into an appropriate position, the roller is again rotated in the predetermined direction (the direction of the arrow), whereby the stent-accommodating tube body 5 is moved toward the proximal end, and the stent 10 is exposed from the distal opening of the stent-accommodating tube body 5. The roller is rotated in the predetermined direction (the direction of the arrow) until the proximal end of the stent is exposed, whereby the stent is released completely from the stent-accommodating tube body, and is disengaged from the inner tube body 3. In addition, in the stent delivery system 1 in this embodiment, the liquid-injecting device (not shown) can be connected to the connector 46, and a liquid can be injected into the stent delivery system 1 by the liquid-injecting device thus connected. Besides, the liquid injected via the connector 46 passes through the lumen 38 inside the proximal-side tube 34, and flows out in the distal-side portion of the stent delivery system (the stent-accommodating tube body), whereby the inside of the stent delivery system (the stent-accommodating tube body) can be flushed. Further, the liquid can also be ejected via the distal end of the stent delivery system (the stent-accommodating tube body).

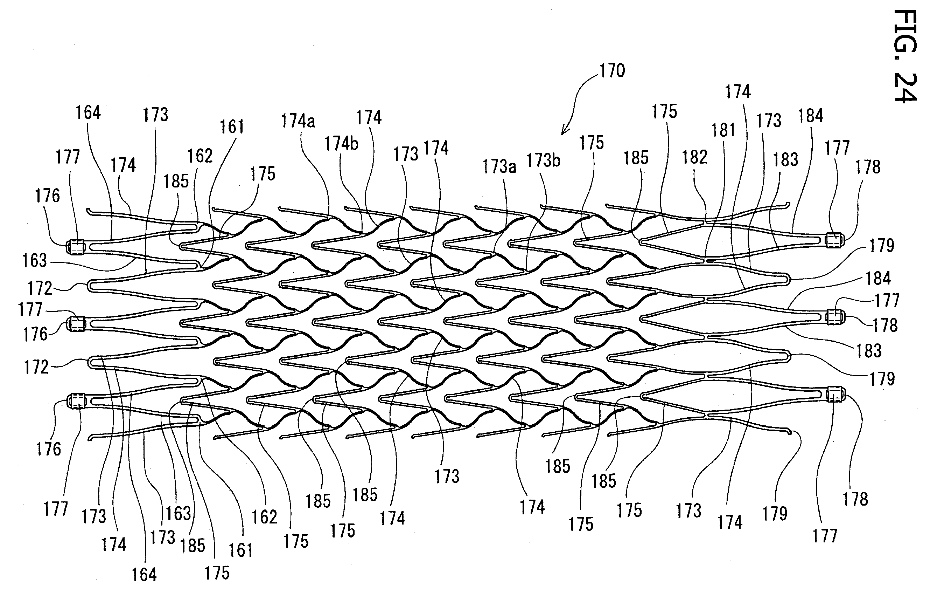

In addition, the stent for use in the present invention may be one as shown in FIG. 24. Like the above-described stent 10, a stent 170 is a so-called self-expandable stent which has a multiplicity of openings in a side surface thereof and is capable of being restored into its pre-compression shape by expanding outward at the time of indwelling in a living body. Further, the stent 170 has a distal end portion oriented toward the distal end of the stent-accommodating tube body (stent-accommodating member) 5 and a proximal end portion oriented toward the proximal end of the stent-accommodating tube body (stent-accommodating member) 5. Furthermore, the stent 170 substantially does not have any bent free end which is at least oriented toward the proximal end, other than the proximal end portion thereof. After the distal end portion of the stent 170 is exposed from the stent-accommodating tube body 5, the exposed distal end portion can be re-accommodated into the stent-accommodating tube body 5 by moving the stent-accommodating tube body 5 toward the distal end relative to the inner tube body 3.

This stent 170 is an in-vivo indwelling stent formed in a roughly cylindrical shape. The stent 170 includes wavy struts 173, 174 extending in the axial direction from one end to the other end of the stent 170 and arranged in plurality along the circumferential direction of the stent, and a plurality of link struts 175 each interconnecting adjacent ones of the wavy struts 173, 174. The adjacent wavy struts 173, 174 have pluralities of close portions and open portions. Each link strut 175 interconnects a close portion of the adjacent wavy struts 173, 174, and is provided at a central portion thereof with a bent section 185 oriented toward the distal end in the axial direction of the stent.

Particularly, in this stent 170, the plurality of wavy struts 173, 174 include a plurality of first wavy struts 173 each having a plurality of upper points 173a and a plurality of lower points 173b, and a plurality of second wavy struts 174 each having a plurality of upper points 174a and a plurality of lower points 174b and each located between the first wavy struts. Each adjacent pair of the first wavy strut 173 and the second wavy strut 174 are so arranged that the upper points or lower points of one of the two wavy struts and the lower points or upper points of the other of the two wavy struts adjacent thereto are set to substantially face each other, thereby forming the close portions. Each link strut 175 interconnects an upper point 173a or a lower point 173b of the first wavy strut 173 and a lower point 174b or an upper point 174a of the second wavy strut which constitute the close portion. In addition, each adjacent pair of the first wavy strut 173 and the second wavy strut 174 are so arranged that the lower points or upper points of one of the two wavy struts and the upper points or lower points of the other of the two wavy struts adjacent thereto are set to substantially face each other, thereby forming the open portions.

This stent 170 is a so-called self-expandable stent which is formed in a roughly cylindrical shape, is compressed toward a center axis thereof at the time of insertion into a living body, and is restored into its pre-compression shape by expanding outward at the time of indwelling in the living body.

The first wavy struts 173 extend in the axial direction substantially parallel to the center axis of the stent. In addition, the first wavy struts 173 are arranged in plurality along the circumferential direction of the stent. The number of the first wavy struts 173 is preferably two or more, particularly preferably three to eight. Further, the plurality of first wavy struts 173 are preferably arranged at substantially regular angular intervals around the center axis of the stent.

Besides, in this stent 170, the first wavy strut 173 has a series of substantially the same waveform over a predetermined length, exclusive of both end portions. Specifically, the first wavy strut 173 has a series of waves having substantially the same waveform, namely, same wavelength and same amplitude, over its portion other than the vicinity of both end portions. In a case where the first wavy strut 173 has the same waveform over substantially the whole part thereof, the wavelength, which varies depending on an outside diameter of the stent, is preferably 0.5 to 8.0 mm, particularly preferably 2.0 to 4.0 mm, and the amplitude is preferably 0.1 to 10.0 mm, particularly preferably 0.3 to 3.0 mm.

The second wavy struts 174 also extend in the axial direction substantially parallel to the center axis of the stent. Besides, the second wavy struts 174 are arranged in plurality along the circumferential direction of the stent, with each of the second wavy struts 174 being arranged between the first wavy struts. The number of the second wavy struts 174 is preferably two or more, particularly preferably three to eight. Further, the plurality of second wavy struts 174 are preferably arranged at substantially regular angular intervals around the center axis of the stent. In addition, the number of the second wavy struts 174 is equal to the number of the first wavy struts.

Besides, in this stent 170, the second wavy strut 174 has a series of substantially the same waveform over a predetermined length, exclusive of both end portions. Specifically, the second wavy strut 174 has a series of waves having substantially the same waveform, namely, same wavelength and same amplitude, over its portion other than the vicinity of both end portions. In a case where the second wavy strut 174 has the same waveform over substantially the whole part thereof, the wavelength, which varies depending on the outside diameter of the stent, is preferably 0.5 to 8.0 mm, particularly preferably 2.0 to 4.0 mm, and the amplitude is preferably 0.1 to 10.0 mm, particularly preferably 0.3 to 3.0 mm.

Furthermore, in this stent 170, the first wavy strut 173 and the second wavy strut 174 are substantially the same in waveform. Specifically, in this stent 170, the first wavy strut 173 and the second wavy strut 174 have substantially the same wavelength and substantially the same amplitude. In addition, the second wavy struts 174 are positionally shifted from the first wavy struts 173 by about a half of the wavelength along the axial direction of the stent.

Consequently, as shown in FIG. 24, the first wavy strut 173 and the second wavy strut 174 adjacent to each other are so situated that the upper points 173a or lower points 173b of the first wavy strut 173 are substantially opposed to the lower points 174b or upper points 174a of the second wavy strut 174, whereby the close portions and the open portions are formed. In other words, in this stent 170, the first wavy strut 173 and the second wavy strut 174 adjacent to each other are so situated that their respective upper portions are not opposed to each other and that their respective lower portions are not opposed to each other; therefore, the close portions and the open portions are provided alternately along the axial direction.

In addition, in the stent in this embodiment, the wavy struts 173, 174 are all the same in length, exclusive of both end portions. Therefore, when the stent is compressed toward the center axis thereof, the struts approach one another in parallel to the axial direction. Besides, since all the wavy struts are the same in length, the stent is favorably compressed radially without stiffing in the axial direction. In addition, in the stent in this embodiment, the wavy struts 173 and 174 are arranged at regular angular intervals around the center axis of the stent, exclusively of both ends thereof. This ensures that, when the stent is compressed toward the center axis thereof, gaps between the struts are reduced in an even manner, so that favorable compression can be achieved without any overlapping of the struts.

Besides, in this stent 170, as shown in FIG. 24, the link struts 175 are provided each of which interconnects the close portions of the adjacent wavy struts 173, 174, and has at its central portion the bent section 185 oriented toward the distal end in the axial direction of the stent. The axial length of the link strut 175, which varies depending on the outside diameter of the stent, is preferably 0.1 to 3.0 mm, particularly preferably 0.5 to 2.0 mm. In addition, the link strut 175 is symmetrical about the center axis of the stent 170 and about the vertex of the bent section 185. Besides, in this stent 170, substantially all the plurality of close portions of the first wavy strut 173 and the second wavy strut 174 adjacent to each other are interconnected by the link struts 175. In addition, the bent section 185 of the link strut 175 is located in the vicinity of the open portion formed between the wavy struts 173, 174. Besides, the bent section 185 of the link strut 175 is a free end oriented toward the distal end of the stent 170. In addition, in the stent 170 in this embodiment, the link struts 175 are disposed in plurality and in series along the axial direction of the stent. Besides, the link struts 175 are disposed in plurality along the circumferential direction of the stent.

In addition, the stent 170 in this embodiment has, at the distal end portion of the stent: bent sections 172 each formed by coupling a distal end portion of the first wavy strut 173 and a distal end portion of the second wavy strut 174; bent sections 176 each formed by coupling a distal end portion of a filamentous part 163 connected to the first wavy strut 173 via a branching section 161 and a distal end portion of a filamentous part 164 connected to the second wavy strut 174 via a branching section 162. The bent sections 172 and the bent sections 176 are disposed alternately along the circumferential direction. Besides, a radiopaque marker 177 is attached to each of the bent sections 176. In addition, the bent sections 176 each having the radiopaque marker 177 are located on the more distal side of the stent than the bent sections 172.

Besides, the stent 170 has, at the proximal end portion of the stent: bent sections 179 each formed by coupling a proximal end portion of the first wavy strut 173 and a proximal end portion of the second wavy strut 174; bent sections 178 each formed by coupling a proximal end portion of a filamentous part 183 connected to the first wavy strut 173 via a branching section 181 and a proximal end portion of a filamentous part 184 connected to the second wavy strut 174 via a branching section 182. The bent sections 179 and the bent sections 178 are disposed alternately along the circumferential direction. In other words, in the stent 170, the bent sections 178 and the bent sections 179 form the proximal end portion oriented toward the proximal end of the stent-accommodating tube body. In addition, the radiopaque marker 177 is attached to each of the bent sections 178. Incidentally, in this stent 170, the radiopaque marker 177 forms a proximal-side inwardly projecting section 177a which will be described later. Besides, the bent sections 178 each having the radiopaque marker 177 are located on the more proximal side of the stent than the bent sections 179. In addition, the stent 170 does not have any free end oriented to the proximal end of the stent, other than the bent sections 178, 179. Therefore, when the stent-accommodating tube body is moved toward the distal end relative to the inner tube body after the distal end portion of the stent is partially exposed from the stent-accommodating tube body, the stent would not be caught on the stent-accommodating tube body since the stent does not have any free end oriented toward the stent-accommodating tube body. Consequently, re-accommodation of the stent into the stent-accommodating tube body (stent-accommodating member) can be achieved. Besides, in this stent 170, the filamentous parts 183 and 184 constituting the bent sections 178 of the proximal-side portion are longer along the axial direction than the filamentous parts 163 and 164 constituting the bent sections 176 of the distal-side portion. The stent 170 is inserted into a living body, starting from the distal end side (the side of the bent sections 176), to be indwelled.

In addition, the radiopaque marker 177 envelops part of or substantially the whole part of two frame sections constituting the bent section. Besides, the radiopaque marker 177 has a thin rectangular parallelepiped shape, houses the two frame sections therein, and is hollowed at a central portion thereof, whereby it is fixed to the two frame sections. As a material for forming the radiopaque marker, one element (simple substance) or two or more elements (alloy) selected from the element group consisting of iridium, platinum, gold, rhenium, tungsten, palladium, rhodium, tantalum, silver, ruthenium, and hafnium can be used suitably. In addition, the length of the marker is preferably 0.1 to 4.0 mm, particularly preferably 0.3 to 1.0 mm. Besides, the thickness of the marker is preferably 0.01 to 0.30 mm, particularly preferably 0.03 to 0.10 mm.

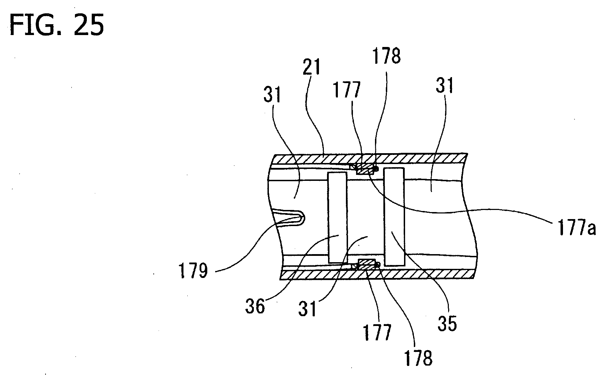

FIG. 25 is an illustration for explaining the stent delivery system in which the in-vivo indwelling stent of FIG. 24 is used.
As shown in FIG. 25, the stent 170 has the proximal-side inwardly projecting section 177a composed of the radiopaque marker 177. In addition, the stent 170 is so disposed that only proximal-side inwardly projecting sections 177a, namely, only the bent sections 178, are located between the distal-side contact section 36 and the proximal-side contact section 35 of the inner tube body 3. Besides, the bent sections 179 each disposed between adjacent bent sections 178 are located on the distal side relative to the distal-side contact section 36. Such a configuration ensures that, when the stent 170 is compressed toward the center axis thereof, the adjacent radiopaque markers 177 are prevented from making contact with each other or overlapping with each other, so that good compression is achieved. In addition, it is also easy to dispose the proximal-side inwardly projecting sections 177a of the stent between the distal-side contact section 36 and the proximal-side contact section 35.

## Claims

1. A stent delivery system (1) comprising:
a stent delivery system main body (2) including
a stent (10) having a multiplicity of sidewall openings, formed in a roughly cylindrical shape, compressed toward a center axis thereof at the time of insertion into a living body, and capable of being restored into its pre-compression shape by expanding outward at the time of indwelling in the living body,
an inner tube body (3) having a guide wire lumen (61), and
a stent-accomodating tube body (5) accommodating said stent (10) in a distal end portion thereof,
said stent (10) being so disposed as to cover a distal end portion of said inner tube body (3), and
said stent (10) being releasable by moving said stent-accommodating tube (5) body in a proximal direction relative to said inner tube body (3) ; and
an operation unit (6) disposed at a proximal end portion of the stent delivery system main body (2) and having a moving mechanism for moving said stent-accommodating tube body,
said inner tube body (3) includes
a distal-side tube (31) having said guide wire lumen (61), and
a proximal-side tube (34) connected to a proximal end side of said distal-side tube (31) and penetrating said stent-accommodating tube body (5),
a wire-formed member (33), interconnecting the proximal end portion of the distal-side tube (31) and the distal end portion of the proximal-side tube (34),
said operation unit (6) includes
a housing (40),
a shaft-like rack member (43) accommodated in said housing (40) and fixed to a proximal end of said stent-accommodating tube body (5),
an operation rotary roller (50) having a working gear wheel (54) which engages with teeth (66) of said rack member (43) so as to move said rack member (43) within said housing (40), and
a connector (46) which is fixed to a proximal end portion of said proximal-side tube (34) protruding beyond the proximal end of said stent-accommodating tube body (5) fixed to said rack member (43) and which is held by said housing (40), and
said stent delivery system (1) has a stent-holding function (35,36) for releasably holding said stent (10) and for enabling re-accommodation of said stent (10) into said stent-accommodating tube body (5) by forward movement of said stent-accommodating tube body (5) after partial exposure of said stent (10) from said stent-accommodating tube body (5), said stent (10) can be released from said stent-accommodating tube body (5) by movement of said rack member (43) toward said connector (46) by rotation of said operation rotary roller (50) in a predetermined direction, and, after partial exposure of said stent (10) from said stent-accommodating tube body (5), said stent (10) can be re-accommodated into said stent-accommodating tube body (5) by moving said rack member (43) within said housing (40) in a direction opposite to a direction toward said connector (46) through rotating said operation rotary roller (50) in a direction reverse to the predetermined **characterized in that** the stent-accommodating tube body (5) includes a distal tube (21) and a proximal tube (22) fixed to a proximal end of the distal tube 21, wherein the distal tube (21) is more flexible than the proximal tube (22, wherein said operation unit (6) has a lock mechanism by which said rack member (43) is releasably locked and a roller intermittent rotation mechanism which imparts a rotation resistance to said operation rotary roller (50) and enables intermittent rotation of the roller.

2. The stent delivery system (1) according to claim 1, wherein said proximal-side tube (34) has a lumen (38) a distal end portion of which opens in said stent-accommodating tube body (5) and which provides communication to a proximal end of said proximal-side tube (34).

3. The stent delivery system (1) according to claim 2, wherein liquid can be injected into said stent (1) delivery system from said connector (46) by using said lumen (38) inside said proximal-side tube.

4. The stent delivery system (1) according to any one of claims 1 to 3, wherein said stent (10) includes a distal end portion oriented toward a distal end of said stent-accommodating tube body (5) and a proximal end portion oriented toward the proximal end of said stent-accommodating tube body (5), said stent (10) does not have any proximally oriented bent free end other than the proximal end portion thereof, and, after exposure of a distal-side portion from said stent-accommodating tube body (5), the exposed portion can be re-accommodated into said stent-accommodating tube body (5) by moving said stent-accommodating tube body (5).

5. The stent delivery system (1) according to any one of claims 1 to 4, wherein said operation unit (6) has a movement-restraining section which, at the time of rotation of said operation rotary roller (50) in said predetermined direction, makes contact with an end portion of said rack member (43) to thereby restrain said rack member (43) from being moved in excess of a predetermined extent.

6. The stent delivery system (1) according to any one of claims 1 to 5, wherein said operation unit (6) has the movement-restraining section which, at the time of rotation of said operation rotary roller (50) in the direction reverse to said predetermined direction, makes contact with an end portion of said rack member to thereby restrain said rack from being moved in excess of a predetermined extent.

7. The stent delivery system (1) according to any one of claims 1 to 6, wherein said stent delivery system has, as said stent-holding function (35,36), a distal-side contact section (36) as such a portion of said inner tube body (3) as to be located inside the proximal end portion of said stent (10) and as not to enter said sidewall openings of said stent (10), and a proximal-side contact section (35) as such a portion of said inner tube body (3) as to be located rearward of the proximal end of said stent (10) in proximity to said distal-side contact section (36), the proximal-side contact section (35) being able to make contact with the proximal end of said stent (10).

8. The stent delivery system (1) according to claim 7, wherein as said stent-holding function (35,36) of said stent delivery system (1), said stent (10) has a proximal-side inwardly projecting section capable of making contact with said distal-side contact section (36), said proximal-side inwardly projecting section being located between said distal-side contact section (36) and said proximal-side contact section (35) of said inner tube body (3).

9. The stent delivery system (1) according to any one of claims 1 to 6, wherein said stent delivery system (1) has, as said stent-holding function (35,36), an elastic member (85) which is disposed on said inner tube body (3) at such a position as to be at least in the proximal end portion of said stent (10) and which presses said stent (10) toward said stent-accommodating tube body (5), and said stent (10) is gripped between said elastic member (85) and said stent-accommodating tube body (5) and is slidable relative to said stent-accommodating tube body (5).

## Patentansprüche

1. Stent-Einführsystem (1), umfassend:
einen Hauptkörper (2) des Stent-Einführsystems, beinhaltend
einen Stent (10), die eine Vielzahl von Seitenwand-Öffnungen aufweist, in einer ungefähr zylindrischen Form ausgebildet ist, und zum Zeitpunkt des Einführens in einen lebenden Körper zu einer zentralen Achse davon zusammengedrückt ist, und imstande ist, zum Zeitpunkt des Verweilens im lebenden Körper durch Expansion in ihre Form vor dem Zusammendrücken zurückversetzt zu werden,
einen inneren Rohrkörper (3), welcher ein Führungsdraht-Lumen (61) aufweist, und
einen Stent-aufnehmenden Rohrkörper (5), welcher den Stent (10) in einem distalen Endabschnitt davon aufnimmt,
wobei der Stent (10) so angeordnet ist, dass sie einen distalen Endabschnitt des inneren Rohrkörpers (3) bedeckt, und
der Stent (10) freisetzbar ist, indem der Stent-aufnehmende Rohrkörper (5) in eine proximale Richtung relativ zum inneren Rohrkörper (3) bewegt wird; und
eine Bedienungseinheit (6), welche an einem proximalen Endabschnitt des Hauptkörpers (2) des Stent-Einführsystems angeordnet ist und einen Bewegungsmechanismus besitzt, um den Stent-aufnehmenden Rohrkörper zu bewegen,
wobei der innere Rohrkörper (3) ein Rohr (31) auf einer distalen Seite, welches das Führungsdraht-Lumen (61) aufweist, und
ein Rohr (34) auf einer proximalen Seite, das mit einer proximalen Endseite vom Rohr (31) auf einer distalen Seite verbunden ist und durch den Stent-aufnehmenden Rohrkörper (5) durchgeht, einschließt,
ein drahtförmig ausgebildetes Teil (33), welches den proximalen Endabschnitt des Rohrs (31) auf der distalen Seite und den distalen Endabschnitt des Rohrs auf der proximalen Seite (41) verbindet,
wobei besagte Operationseinheit (6) einschließt:
ein Gehäuse (40),
ein schaftartiges Zahnstangen-Teil (43), welches im Gehäuse (40) aufgenommen ist und an einem proximalen Ende des Stent-aufnehmenden Rohrkörpers (5) befestigt ist,
einen Bedienungsdrehroller (50), welcher ein Arbeits-Zahnrad (54) besitzt, welches mit den Zähnen (66) des Zahnstangenteils (43) in Eingriff steht, um das Zahnstangenteil (43) innerhalb des Gehäuses (40) zu bewegen, und
ein Verbindungsteil (46), welches an einem proximalen Endabschnitt vom Rohr (34) auf der proximalen Seite befestigt ist und über das proximale Ende des Stent-aufnehmenden Rohrkörpers (5) hinaussteht, welches am Zahnstangenteil (43) befestigt ist, und welches durch das Gehäuse (40) gehalten wird, und
wobei das Stent-Einführsystem (1) eine Stent-haltende Funktion (35, 36) besitzt, um den Stent (10) freisetzbar festzuhalten und um eine Wiederaufnahme des Stent (10) in den Stent-aufnehmende Rohrkörper (5) durch Vorwärtsbewegen des Stent-aufnehmende Rohrkörpers (5) nach partieller Freisetzung des Stent (10) aus dem Stent-aufnehmenden Rohrkörper (5) zu ermöglichen, wobei der Stent (10) vom Stent-aufnehmenden Rohrkörper (5) durch Bewegen des Zahnstangenteils (43) hin zum Verbindungsteil (46) durch Rotation von besagtem Bedienungsdrehroller (50) in eine vorbestimmte Richtung freigesetzt werden kann, und, nach partiellem Bloßlegen des Stent (10) aus dem Stent-aufnehmenden Rohrkörper (5), der Stent (10) in den Stent-aufnehmenden Rohrkörper (5) durch Bewegen vom Zahnstangenteil (43) innerhalb vom Gehäuse (40) in einer zur Richtung hin zum Verbindungsteil (46) entgegengesetzten Richtung durch Drehen vom Bedienungsdrehroller (50) in einer Richtung rückwärts zur vorbestimmten Richtung wieder aufgenommen werden kann, **dadurch gekennzeichnet, dass** der Stent-aufnehmende Rohrkörper (5) ein distales Rohr (21) und ein proximales Rohr (22), welches an einem proximalen Ende vom distalen Rohr (21) befestigt ist, einschließt, wobei das distale Rohr (21) flexibler ist als das proximale Rohr (22), wobei die Bedienungseinheit (6) einen Verriegelungsmechanismus, durch welchen das Zahnstangenteil (43) wieder lösbar verriegelt ist, und einen Roller-unterbrechenden Drehmechanismus, welcher dem Bedienungsdrehroller (50) einen Rotationswiderstand auferlegt und die diskontinuierliche Drehung des Rollers ermöglicht, besitzt.

2. Stent-Einführsystem (1) gemäß Anspruch 1, wobei das Rohr (34) auf der proximalen Seite ein Lumen (38) aufweist, dessen distaler Endabschnitt sich in den Stent-aufnehmenden Rohrkörper (5) öffnet und welches in Kommunikation mit einem proximalen Ende vom Rohr (34) auf proximaler Seite steht.

3. Stent-Einführsystem (1) gemäß Anspruch 2, wobei Flüssigkeit vom Verbindungsteil (46) in das Stent-Einführsystem (1) durch Verwendung des Lumens (38) innerhalb des Rohrs auf der proximalen Seite injiziert werden kann.

4. Stent-Einführsystem (1) gemäß einem der Ansprüche 1 bis 3, wobei der Stent (10) einen distalen Endabschnitt, welcher hin zu einem distalen Ende von dem Stent-aufnehmenden Rohrkörper (5) orientiert ist, und einen proximalen Endabschnitt, welcher hin zum proximalen Ende von dem Stent-aufnehmenden Rohrkörper (5) orientiert ist, einschließt, wobei der Stent (10) kein proximal orientiertes gebogenes freies Ende außer dem proximalen Endabschnitt davon besitzt, und wobei nach Bloßlegen eines Abschnitts auf der distalen Seite des Stent-aufnehmenden Rohrkörpers (5) der bloßgelegte Abschnitt in den Stent-beinhaltenden Rohrkörper (5) durch Bewegen des Stent-aufnehmenden Rohrkörpers (5) wieder aufgenommen werden kann.

5. Stent-Einführsystem (1) gemäß einem der Ansprüche 1 bis 4, wobei die Bedienungseinheit (6) einen Abschnitt zum Erschweren einer Bewegung aufweist, welcher zum Zeitpunkt der Drehung vom Bedienungsdrehroller (50) in der vorbestimmten Richtung mit einem Endabschnitt des Zahnstangenteils (43) in Kontakt tritt, um dabei das Zahnstangenteil (43) davon abzuhalten, über ein vorbestimmtes Ausmaß hinaus bewegt zu werden.

6. Stent-Einführsystem (1) gemäß einem der Ansprüche 1 bis 5, wobei die Operationseinheit (6) den Abschnitt zum Erschweren einer Bewegung aufweist, welcher zum Zeitpunkt der Drehung vom Bedienungsdrehroller (50) in die rückwärts zur vorbestimmten Richtung gerichtete Richtung in Kontakt mit einem Endabschnitt des Zahnstangenteils tritt, um dabei das Zahnstangenteil (43) davon abzuhalten, über ein vorbestimmtes Ausmaß hinaus bewegt zu werden.

7. Stent-Einführsystem (1) gemäß einem der Ansprüche 1 bis 6, wobei das Stent-Einführsystem als Stent-haltende Funktion (35, 36) einen Kontaktabschnitt (36) auf einer distalen Seite als einen Abschnitt des inneren Rohrkörpers (3), um innerhalb des proximalen Endabschnitts des Stent (10) positioniert zu sein und nicht in die Seitenwand-Öffnungen des Stent (10) einzudringen, und einen Kontaktabschnitt (35) auf proximaler Seite als einen Abschnitt des inneren Rohrkörpers (3) um rückwärts gerichtet des proximalen Endabschnitts des Stent (10) in Nähe des Kontaktabschnitts (36) auf distaler Seite positioniert zu sein, aufweist, wobei der Kontaktabschnitt (35) auf proximaler Seite dazu in der Lage ist, mit dem proximalen Ende der Stent (10) in Kontakt zu stehen.

8. Stent-Einführsystem (1) gemäß Anspruch 7, wobei die Stent (10) als Stent-haltende Funktion (35, 36) des Stent-Einführsystems (1) einen nach innen hineinstehenden Abschnitt auf proximaler Seite aufweist, welcher dazu in der Lage ist, mit dem Kontaktabschnitt (36) auf distaler Seite in Kontakt zu stehen, wobei der nach innen hervorstehende Abschnitt auf proximaler Seite zwischen dem Kontaktabschnitt (36) auf distaler Seite und dem Kontaktabschnitt (35) auf proximaler Seite des inneren Rohrkörpers (3) angeordnet ist.

9. Stent-Einführsystem (1) gemäß einem der Ansprüche 1 bis 6, wobei das Stent-Einführsystem (1) als Stent-haltende Funktion (35, 36) ein elastisches Teil (85) aufweist, welches auf dem inneren Rohrkörper (3) auf solch einer Position angeordnet ist, so dass es mindestens auf dem proximalen Endabschnitt der Stent (10) angeordnet ist und welches den Stent (10) zum Stent-aufnehmenden Rohrkörper (5) drückt, und wobei die Stent (10) zwischen dem elastischen Bauteil (85) und dem Stent-aufnehmenden Rohrkörper (5) festgehalten wird und relativ zum Stent-aufnehmenden Rohrkörper (5) gleitbar ist.

## Revendications

1. Dispositif de pose d'endoprothèse (1) comprenant :
un corps principal de dispositif de pose d'endoprothèse (2) comportant :
une endoprothèse (10) comprenant un certain nombre d'ouvertures de paroi latérale, réalisée globalement de forme cylindrique, compressée vers un axe central de celle-ci au moment de l'insertion dans un corps vivant, et pouvant être ramenée à sa forme d'avant compression par dilatation externe au moment de l'introduction dans le corps vivant,
un corps tubulaire interne (3) comprenant une lumière de fil de guidage (61), et
un corps tubulaire de réception d'endoprothèse (5) recevant ladite endoprothèse (10) dans une partie d'extrémité distale de celui-ci,
ladite endoprothèse (10) étant disposée de manière à recouvrir une partie d'extrémité distale dudit corps tubulaire interne (3), et
ladite endoprothèse (10) pouvant être libérée en déplaçant ledit corps tubulaire de réception d'endoprothèse (5) dans une direction proximale par rapport audit corps tubulaire interne (3) ; et
une unité d'activation (6) disposée au niveau d'une partie d'extrémité proximale du corps principal de dispositif de pose d'endoprothèse (2) et comprenant un mécanisme de déplacement destiné à déplacer le corps tubulaire de réception d'endoprothèse,
ledit corps tubulaire interne (3) comporte :
un tube du côté distal (31) comprenant une lumière de fil de guidage (61), et
un tube du côté proximal (34) raccordé à un côté d'extrémité proximale dudit tube du côté distal (31) et pénétrant dans ledit corps tubulaire de réception d'endoprothèse (5),
un élément en forme de fil (33), reliant ensemble la partie d'extrémité proximale du tube du côté distal (31) et la partie d'extrémité distale du tube du côté proximal (34),
ladite unité d'activation (6) comporte :
un boîtier (40),
un élément de crémaillère en forme d'arbre (43) reçu dans ledit boîtier (40) et fixé à une extrémité proximale dudit corps tubulaire de réception d'endoprothèse (5),
un rouleau d'activation tournant (50) comprenant une roue dentée active (54) qui est couplée à des dents (66) dudit élément de crémaillère (43) afin de déplacer ledit élément de crémaillère (43) à l'intérieur dudit boîtier (40), et
un coupleur (46) qui est fixé sur une partie d'extrémité proximale dudit tube du côté proximal (34) s'étendant au-delà de l'extrémité proximale dudit corps tubulaire de réception d'endoprothèse (5) fixé sur ledit élément de crémaillère (43) et qui est maintenu par ledit boîtier (40), et
ledit dispositif de pose d'endoprothèse (1) présente une fonction de retenue d'endoprothèse (35, 36) pour retenir de manière amovible ladite endoprothèse (10) et de permettre la remise en place de ladite endoprothèse (10) dans ledit corps tubulaire de réception d'endoprothèse (5) par un mouvement vers l'avant dudit corps tubulaire de réception d'endoprothèse (5) après exposition partielle de ladite endoprothèse (10) à partir dudit corps tubulaire de réception d'endoprothèse (5), ladite endoprothèse (10) peut être libérée dudit corps tubulaire de réception d'endoprothèse (5) par un mouvement dudit élément de crémaillère (43) vers ledit coupleur (46) par rotation dudit rouleau d'activation tournant (50) dans un sens prédéterminé, et, après exposition partielle de ladite endoprothèse (10) depuis ledit corps tubulaire de réception d'endoprothèse (5), ladite endoprothèse (10) peut être remise en place dans ledit corps tubulaire de réception d'endoprothèse (5) en déplaçant ledit élément de crémaillère (43) à l'intérieur dudit boîtier (40) dans un sens opposé au sens se rapprochant dudit coupleur (46) par la rotation dudit rouleau d'activation tournant (50) dans un sens inverse au sens prédéterminé, **caractérisé en ce que** le corps tubulaire de réception d'endoprothèse (5) comporte un tube distal (21) et un tube proximal (22) fixé sur une extrémité proximale du tube distal (21), dans lequel le tube distal (21) est plus flexible que le tube proximal (22),
dans lequel ladite unité d'activation (6) comporte un mécanisme de verrouillage par lequel ledit élément de crémaillère (43) est verrouillé de manière amovible et un mécanisme de rotation intermittent de rouleau qui communique une résistance à la rotation audit rouleau d'activation tournant (50) et permet la rotation intermittente du rouleau.

2. Dispositif de pose d'endoprothèse (1) selon la revendication 1, dans lequel ledit tube du côté proximal (34) comporte une lumière (38) dont une partie d'extrémité distale débouche dans ledit corps tubulaire de réception d'endoprothèse (5) et qui assure la communication avec une extrémité proximale dudit tube du côté proximal (34).

3. Dispositif de pose d'endoprothèse (1) selon la revendication 2, dans lequel un liquide peut être injecté dans ledit dispositif de pose d'endoprothèse (1) à partir dudit coupleur (46) en utilisant ladite lumière (38) à l'intérieur dudit tube du côté proximal.

4. Dispositif de pose d'endoprothèse (1) selon l'une quelconque des revendications 1 à 3, dans lequel ladite endoprothèse (10) comporte une partie d'extrémité distale orientée vers une extrémité distale dudit corps tubulaire de réception d'endoprothèse (5) et une partie d'extrémité proximale orientée vers l'extrémité proximale dudit corps tubulaire de réception d'endoprothèse (5), ladite endoprothèse (10) ne comportant pas d'extrémité libre courbe, orientée de manière proximale, autre que la partie d'extrémité proximale de celle-ci, et, après exposition d'une partie du côté distal à partir dudit corps tubulaire de réception d'endoprothèse (5), la partie exposée peut être remise en place dans ledit corps tubulaire de réception d'endoprothèse (5) en déplaçant ledit corps tubulaire de réception d'endoprothèse (5).

5. Dispositif de pose d'endoprothèse (1) selon l'une quelconque des revendications 1 à 4, dans lequel ladite unité d'activation (6) comporte une section de limitation de mouvement qui, au moment de la rotation dudit rouleau d'activation tournant (50) dans ledit sens prédéterminé, entre en contact avec une partie d'extrémité dudit élément de crémaillère (43) limitant ainsi le déplacement dudit élément de crémaillère (43) au-delà d'une distance prédéterminée.

6. Dispositif de pose d'endoprothèse (1) selon l'une quelconque des revendications 1 à 5, dans lequel ladite unité d'activation (6) comporte une section de limitation de mouvement qui, au moment de la rotation dudit rouleau d'activation tournant (50) dans le sens inverse par rapport audit sens prédéterminé, entre en contact avec une partie d'extrémité dudit élément de crémaillère limitant ainsi le déplacement dudit élément de crémaillère au-delà d'une distance prédéterminée.

7. Dispositif de pose d'endoprothèse (1) selon l'une quelconque des revendications 1 à 6, dans lequel ledit dispositif de pose d'endoprothèse, comporte, pour ladite fonction de retenue d'endoprothèse (35, 36), une section de contact du côté distal (36) telle qu'une partie dudit corps tubulaire interne (3) de manière à être située à l'intérieur de la partie d'extrémité proximale de ladite endoprothèse (10) et à ne pas entrer dans lesdites ouvertures de paroi latérale de ladite endoprothèse (10), et une section de contact du côté proximal (35) telle qu'une partie dudit corps tubulaire interne (3) de manière à être située vers l'arrière de l'extrémité proximale de ladite endoprothèse (10) à proximité de ladite section de contact du côté distal (36), la section de contact du côté proximal (35) pouvant entrer en contact avec l'extrémité proximale de ladite endoprothèse (10).

8. Dispositif de pose d'endoprothèse (1) selon la revendication 7, dans lequel, pour ladite fonction de retenue d'endoprothèse (35, 36) dudit dispositif de pose d'endoprothèse (1), ladite endoprothèse (10) comporte une section s'étendant vers l'intérieur du côté proximal pouvant entrer en contact avec ladite section de contact du côté distal (36), ladite section s'étendant vers l'intérieur du côté proximal étant située entre ladite section de contact du côté distal (36) et ladite section de contact du côté proximal (35) dudit corps tubulaire interne (3).

9. Dispositif de pose d'endoprothèse (1) selon l'une quelconque des revendications 1 à 6, dans lequel ledit_{┬} dispositif de pose d'endoprothèse (1) comporte, pour ladite fonction de retenue d'endoprothèse (35, 36), un élément élastique (85) qui est disposé sur ledit corps tubulaire interne (3) à une position telle qu'il peut être au moins dans la partie d'extrémité proximale de ladite endoprothèse (10) et qui presse ladite endoprothèse (10) vers ledit corps tubulaire de réception d'endoprothèse (5), et ladite endoprothèse (10) est saisie entre ledit élément élastique (85) et ledit corps tubulaire de réception d'endoprothèse (5) et peut être glissée par rapport audit corps tubulaire de réception d'endoprothèse (5).
